# EUROPEAN PATENT APPLICATION

(11) **EP 3 653 629 A1**
(43) Date of publication of application: **20.05.2020**
(21) Application number: 18206725.6
(22) Date of filing: 16.11.2018
(51) Int. Cl.: C07D 498/18, A61K 31/553, A61P 31/18

(54) **ACID ADDITION SALTS OF AN INTEGRASE STRAND TRANSFER INHIBITOR**

(71) Applicant: Sandoz AG, 4056 Basel (CH)
(72) Inventor: The designation of the inventor has not yet been filed
(74) Representative: Kluschanzoff, Harald

(57) **Abstract**

The present invention relates to acid addition salts of the integrase strand transfer inhibitor bictegravir with acids selected from the group consisting of hydrochloric acid and 1,5 naphthalenedisulfonic acid and crystalline forms thereof. Furthermore, the invention relates to processes for the preparation of the salts and their respective crystalline forms and to a pharmaceutical composition comprising one or more of them, preferably in a predetermined and/or effective amount, at least one pharmaceutically acceptable excipient and optionally one ore more additional antiviral agent(s). The pharmaceutical composition of the present invention can be used as a medicament, in particular for the treatment and/or prophylaxis of HIV-1 infections.

## Description

### FIELD OF THE INVENTION

The present invention relates to acid addition salts of the integrase strand transfer inhibitor bictegravir with acids selected from the group consisting of hydrochloric acid and 1,5 naphthalenedisulfonic acid, and to crystalline forms thereof. Furthermore, the invention relates to processes for the preparation of the salts and their respective crystalline forms and to a pharmaceutical composition comprising one or more of them, preferably in a predetermined and/or effective amount, at least one pharmaceutically acceptable excipient and optionally one ore more additional antiviral agent(s). The pharmaceutical composition of the present invention can be used as a medicament, in particular for the treatment and/or prophylaxis of HIV-1 infections.

### BACKGROUND OF THE INVENTION

Bictegravir is a human immunodeficiency virus type 1 (HIV-1) integrase strand transfer inhibitor (INSTI). A fixed-dose combination of bictegravir and nucleoside reverse transcriptase inhibitors (NRTIs) tenofovir alafenamide and emtricitabine was approved in February, 2018 in the US and in June, 2018 in the EU for the treatment of HIV-1 infection. The drug product is marketed under the brand Biktarvy™. Bictegravir can be chemically designated as (2R,5S,13aR)-7,9-dioxo-10-((2,4,6-trifluorobenzyl)carbamoyl)-2,3,4,5,7,9,13,13a-octahydro-2,5-methanopyrido [1',2':4,5]pyrazino[2,1-b][1,3]oxazepin-8-olate (IUPAC name) or (2R,5S,13aR)-8-hydroxy-7,9-dioxo-*N*-(2,4,6-trifluorobenzyl)-2,3,4,5,7,9,13,13a-octahydro-2,5-methanopyrido [1',2':4,5]pyrazino[2,1-b][1,3]oxazepine-10 carboxamide and is represented by the following chemical structure according to Formula (I)

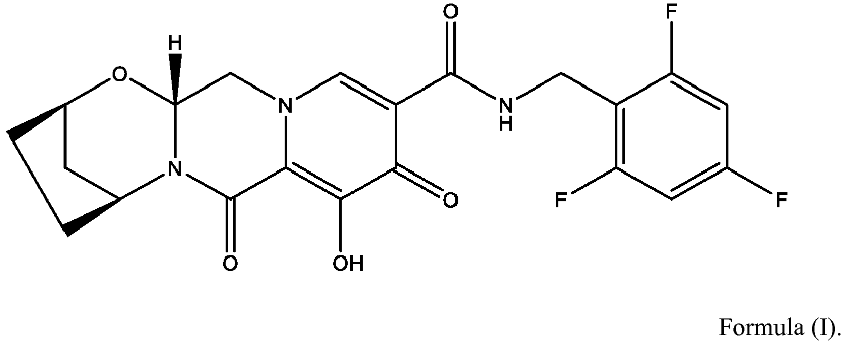

WO 2014/100323 A1 discloses polycyclic carbamoylpyridone compounds and pharmaceutically acceptable salts thereof. Bictegravir is prepared in Example 42 and obtained as the free compound.

WO 2015/196116 A1 and WO 2015/196137 A1 both disclose a crystalline form of bictegravir sodium, designated as "Form I". The commercial product Biktarvy™ contains bictegravir sodium. According to the information provided in the European Public Assessment Report (EPAR) only one polymorphic form of bictegravir sodium has been identified.

WO 2015/196137 A1 discloses various crystalline forms of bictegravir free compound and of bictegravir potassium. The same application discloses that bictegravir forms co-crystals with citric acid, oxalic acid and fumaric acid. According to paragraphs [0290] - [0292] the co-crystals are all prepared by solvent evaporation, a method which is not suitable for industrial scale.

Different solid-state forms of an active pharmaceutical ingredient often possess different properties. Differences in the physicochemical properties of solid-state forms can be important for the improvement of pharmaceutical compositions, for example, pharmaceutical formulations with improved dissolution profile or with improved stability or shelf-life can become accessible due to an improved solid-state form of an active pharmaceutical ingredient. Also processing or handling of the active pharmaceutical ingredient during the formulation process may be improved. New solid-state forms of an active pharmaceutical ingredient can thus have desirable processing properties. They can be easier to handle, better suited for storage, and/or allow for better purification, compared to previously known solid-state forms.

Besides the need for suitable physicochemical properties, solid-state forms intended for pharmaceutical use must also be reliably producible on industrial scale, preferably by using standard manufacturing equipment. Ideally the product is obtainable in high yield.

There remains thus a need for the provision of improved solid-state forms of bictegravir which are reliably producible on industrial scale and which possess physicochemical properties allowing for the reliable production and stable storage of pharmaceutical compositions comprising bictegravir.

### SUMMARY OF THE INVENTION

The present invention provides bictegravir acid addition salts, in particular salts of bictegravir with acids selected from 1,5-naphtalene disulfonic acid and hydrochloric acid. In particular the present invention concerns crystalline bictegravir *hemi*-napadisylate and crystalline bictegravir mono-hydrochloride. The salts of the present invention are physically and chemically stable against moisture and/or temperature stress, are non-hygroscopic and due to their morphology exhibit excellent powder properties regarding compactibility, bulk density and flowability. In addition, they can be reliably produced on industrial scale and in high yield.

### Abbreviations

- PXRD: powder X-ray diffractogram
- FTIR: Fourier transform infrared
- ATR: attenuated total reflection
- DSC: differential scanning calorimetry
- TGA: thermogravimetric analysis
- RH: relative humidity
- GMS: gravimetric moisture sorption

### Definitions

In the context of the present invention the following definitions have the indicated meaning, unless explicitly stated otherwise:
As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% relative humidity, preferably 30-70% relative humidity, more preferably 40-60% relative humidity and most preferably 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials " by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 6.5° 2-Theta for example can appear between 6.3° and 6.7° 2-Theta, preferably between 6.4 and 6.6° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "essentially the same" with reference to Fourier transform infrared spectroscopy means that variabilities in peak positions and relative intensities of the peaks are to be taken into account. For example, a typical precision of the wavenumber values is in the range of ± 2 cm⁻¹. Thus, a peak at 3055 cm⁻¹ for example can appear in the range of from 3053 to 3057 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative peak intensities should therefore be taken as qualitative measure only.

The bictegravir salts of the present invention may be referred to herein as being characterized by a powder X-ray diffractogram or an FTIR spectrum "as shown in" a figure. The person skilled in the art understands that factors such as variations in instrument type, response and variations in sample directionality, sample concentration, sample purity, sample history and sample preparation may lead to variations, for example relating to the exact reflection and peak positions and their intensities. However, a comparison of the graphical data in the figures herein with the graphical data generated for an unknown physical form and the confirmation that two sets of graphical data relate to the same crystal form is well within the knowledge of a person skilled in the art.

The term "solid-state form" as used herein refers to any crystalline and/or amorphous phase of a compound.

The term "anhydrous salt" as used herein refers to a crystalline salt where no water is cooperated in or accommodated by the crystal structure. An anhydrous salt may still contain residual water, which is not part of the crystal structure but may be adsorbed on the surface or absorbed in disordered regions of the crystal. Typically, an anhydrous salt does not contain more than 2.0 w-% of water based on the weight of the salt.

The term "non-solvated salt" as used herein refer to a crystalline salt where no organic solvent is cooperated in or accommodated by the crystal structure. A non-solvated salt may still contain residual organic solvent, which is not part of the crystal structure but may be adsorbed on the surface or absorbed in disordered regions of the crystal. Typically, a non-solvated salt does not contain more than 2.0 w-%, preferably not more than 0.5 w-% of organic solvent based on the weight of the salt.

The term "non-hygroscopic" as used herein refers to a compound showing a water uptake of at most 2 w-% in the sorption cycle when measured with GMS at a relative humidity in the range of from 0 to 90% RH and a temperature of (25.0 ± 0.1) °C based on the weight of the compound.

As used herein, the term "mother liquor" refers to the solution remaining after crystallization of a solid from said solution.

The term "lath-shaped" as used herein with regards to crystal shape refers to elongated, thin and blade-like crystals.

A "predetermined amount" as used herein with regard to a bictegravir salt of the present invention refers to the initial amount of the bictegravir salt used for the preparation of a pharmaceutical composition having a desired dosage strength of bictegravir.

The term "effective amount" as used herein with regard to a bictegravir salt of the present invention encompasses an amount of the bictegravir salt, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient.

### BRIEF DESCRIPTION OF THE FIGURES

**Figure 1****:** illustrates a representative PXRD of the bictegravir *hemi*-napadisylate of the present invention. The x-axis shows the scattering angle in °2-Theta, the y-axis shows the intensity of the scattered X-ray beam in counts of detected photons.
**Figure 2****:** illustrates a representative FTIR spectrum of the bictegravir *hemi*-napadisylate of the present invention. The x-axis shows the wavenumbers in cm⁻¹, the y-axis shows the relative intensity in percent transmittance.
**Figure 3****:** illustrates a representative DSC curve of the bictegravir *hemi*-napadisylate of the present invention. The x-axis shows the temperature in degree Celsius (°C), the y-axis shows the heat flow rate in Watt per gram (W/g) with endothermic peaks going up.
**Figure 4****:** illustrates a representative TGA curve of the bictegravir *hemi*-napadisylate of the present invention. The x-axis shows the temperature in degree Celsius (°C), the y-axis shows the mass (loss) of the sample in weight percent (weight%).
**Figure 5****:** illustrates a representative moisture sorption curve of the bictegravir *hemi-*napadisylate of the present invention in the range of from 0 to 90% relative humidiy. The x-axis displays the relative humidity in percent (%) measured at a temperature of (25.0 ± 0.1) °C, the y-axis displays the equilibrium mass change in weight percent (w-%). The values are displayed as uncorrected values.
**Figure 6****:** illustrates a scanning electron microscopic image of the bictegravir *hemi-*napadisylate of the present invention (magnification: x 500; scale bar: 200 micrometer)
**Figure 7****:** illustrates a representative PXRD of the bictegravir *mono*-hydrochloride of the present invention. The x-axis shows the scattering angle in °2-Theta, the y-axis shows the intensity of the scattered X-ray beam in counts of detected photons.
**Figure 8****:** illustrates a representative FTIR spectrum of the bictegravir *mono*-hydrochloride of the present invention. The x-axis shows the wavenumbers in cm⁻¹, the y-axis shows the relative intensity in percent transmittance.
**Figure 9****:** illustrates a representative DSC curve of the bictegravir *mono*-hydrochloride of the present invention. The x-axis shows the temperature in degree Celsius (°C), the y-axis shows the heat flow rate in Watt per gram (W/g) with endothermic peaks going up.
**Figure 10****:** illustrates a representative TGA curve of the bictegravir *mono*-hydrochloride of the present invention. The x-axis shows the temperature in degree Celsius (°C), the y-axis shows the mass (loss) of the sample in weight percent (weight%).
**Figure 11****:** illustrates a representative moisture sorption curve of the bictegravir *mono-*hydrochloride of the present invention in the range of from 0 to 90% relative humidiy. The x-axis displays the relative humidity in percent (%) measured at a temperature of (25.0 ± 0.1) °C, the y-axis displays the equilibrium mass change in weight percent (w-%). The values are displayed as uncorrected values.
**Figure 12****:** illustrates a scanning electron microscopic image of the bictegravir *mono-*hydrochloride of the present invention (magnification: x 500; scale bar: 200 micrometer)

### DETAILED DESCRIPTION OF THE INVENTION

In a first aspect, the present invention relates to a salt comprising bictegravir and 1,5-naphtalenedisulfonic acid. In particular, the present invention relates to a salt comprising bictegravir and 1,5-naphtalenedisulfonic acid characterized by the chemical structure according to Formula (II) 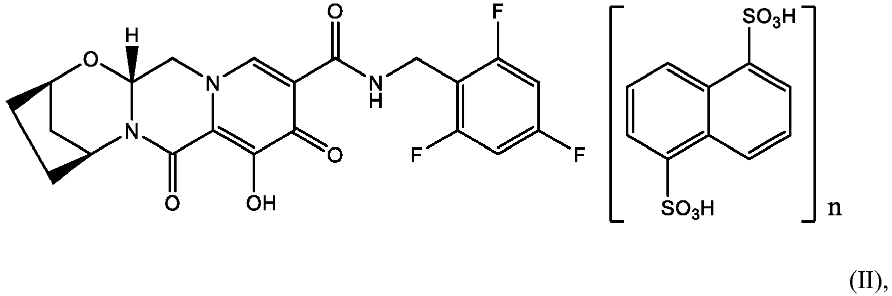 wherein n is in the range of from 0.4 to 0.6, preferably in the range of from 0.45 to 0.55 and most preferably n is about 0.5. For example, the bictegravir salt of the present invention is a bictegravir *hemi*-napadisylate salt e.g. a salt having a molar ratio of bictegravir and 1,5-naphtalenedisulfonic acid in the range of from 1.0: 0.4 to 0.6, preferably in the range of from 1.00: 0.45 to 0.55 and most preferably the molar ratio is about 1.0 to 0.5.

In a particular preferred embodiment, the present invention relates to a salt comprising bictegravir and 1,5-naphtalenedisulfonic acid as defined in any one of the above described embodiments characterized in that the salt is crystalline.

The bictegravir *hemi*-napadisylate salt of the present invention as defined in any one of the above described embodiments may be characterized by analytical methods well known in the field of the pharmaceutical industry for characterizing solids. Such methods comprise but are not limited to powder X-ray diffraction, Fourier transform infrared spectroscopy, differential scanning calorimetry, thermogravimetric analysis and gravimetric moisture sorption. It may be characterized by one of the aforementioned analytical methods or by combining two or more of them. In particular, the bictegravir *hemi*-napadisylate salt of the present invention may be characterized by any one of the following embodiments or by combining two or more of the following embodiments.

The present invention relates to a salt comprising bictegravir and 1,5-naphtalenedisulfonic acid characterized by having a PXRD comprising reflections at 2-Theta angles of:
(7.2 ± 0.2)°, (7.8 ± 0.2)° and (23.5 ± 0.2)°; or
(7.2 ± 0.2)°, (7.8 ± 0.2)°, (20.1 ± 0.2)° and (23.5 ± 0.2)°; or
(7.2 ± 0.2)°, (7.8 ± 0.2)°, (20.1 ± 0.2)°, (21.0 ± 0.2)° and (23.5 ± 0.2)°; or
(7.2 ± 0.2)°, (7.8 ± 0.2)°, (17.2 ± 0.2)°, (20.1 ± 0.2)°, (21.0 ± 0.2)° and (23.5 ± 0.2)°; or
(7.2 ± 0.2)°, (7.8 ± 0.2)°, (14.9 ± 0.2)°, (17.2 ± 0.2)°, (20.1 ± 0.2)°, (21.0 ± 0.2)° and (23.5 ± 0.2)°; or
(6.5 ± 0.2)°, (7.2 ± 0.2)°, (7.8 ± 0.2)°, (14.9 ± 0.2)°, (17.2 ± 0.2)°, (20.1 ± 0.2)°, (21.0 ± 0.2)° and (23.5 ± 0.2)°; or
(6.5 ± 0.2)°, (7.2 ± 0.2)°, (7.8 ± 0.2)°, (11.4 ± 0.2)°, (14.9 ± 0.2)°, (17.2 ± 0.2)°, (20.1 ± 0.2)°, (21.0 ± 0.2)° and (23.5 ± 0.2)°; or
(6.5 ± 0.2)°, (7.2 ± 0.2)°, (7.8 ± 0.2)°, (11.4 ± 0.2)°, (13.1 ± 0.2)°, (14.9 ± 0.2)°, (17.2 ± 0.2)°, (20.1 ± 0.2)°, (21.0 ± 0.2)° and (23.5 ± 0.2)°,
when measured at a temperature in the range of from 20 to 30 °C with Cu-Kalpha_{1,2} radiation having a wavelength of 0.15419 nm.

Alternatively, the present invention relates to a salt comprising bictegravir and 1,5-naphtalenedisulfonic acid characterized by having a PXRD comprising reflections at 2-Theta angles of:
(7.2 ± 0.1)°, (7.8 ± 0.1)° and (23.5 ± 0.1)°; or
(7.2 ± 0.1)°, (7.8 ± 0.1)°, (20.1 ± 0.1)° and (23.5 ± 0.1)°; or
(7.2 ± 0.1)°, (7.8 ± 0.1)°, (20.1 ± 0.1)°, (21.0 ± 0.1)° and (23.5 ± 0.1)°; or
(7.2 ± 0.1)°, (7.8 ± 0.1)°, (17.2 ± 0.1)°, (20.1 ± 0.1)°, (21.0 ± 0.1)° and (23.5 ± 0.1)°; or
(7.2 ± 0.1)°, (7.8 ± 0.1)°, (14.9 ± 0.1)°, (17.2 ± 0.1)°, (20.1 ± 0.1)°, (21.0 ± 0.1)° and (23.5 ± 0.1)°; or
(6.5 ± 0.1)°, (7.2 ± 0.1)°, (7.8 ± 0.1)°, (14.9 ± 0.1)°, (17.2 ± 0.1)°, (20.1 ± 0.1)°, (21.0 ± 0.1)° and (23.5 ± 0.1)°; or
(6.5 ± 0.1)°, (7.2 ± 0.1)°, (7.8 ± 0.1)°, (11.4 ± 0.1)°, (14.9 ± 0.1)°, (17.2 ± 0.1)°, (20.1 ± 0.1)°, (21.0 ± 0.1)° and (23.5 ± 0.1)°; or
(6.5 ± 0.1)°, (7.2 ± 0.1)°, (7.8 ± 0.1)°, (11.4 ± 0.1)°, (13.1 ± 0.1)°, (14.9 ± 0.1)°, (17.2 ± 0.1)°, (20.1 ± 0.1)°, (21.0 ± 0.1)° and (23.5 ± 0.1)°,
when measured at a temperature in the range of from 20 to 30 °C with Cu-Kalpha_{1,2} radiation having a wavelength of 0.15419 nm.

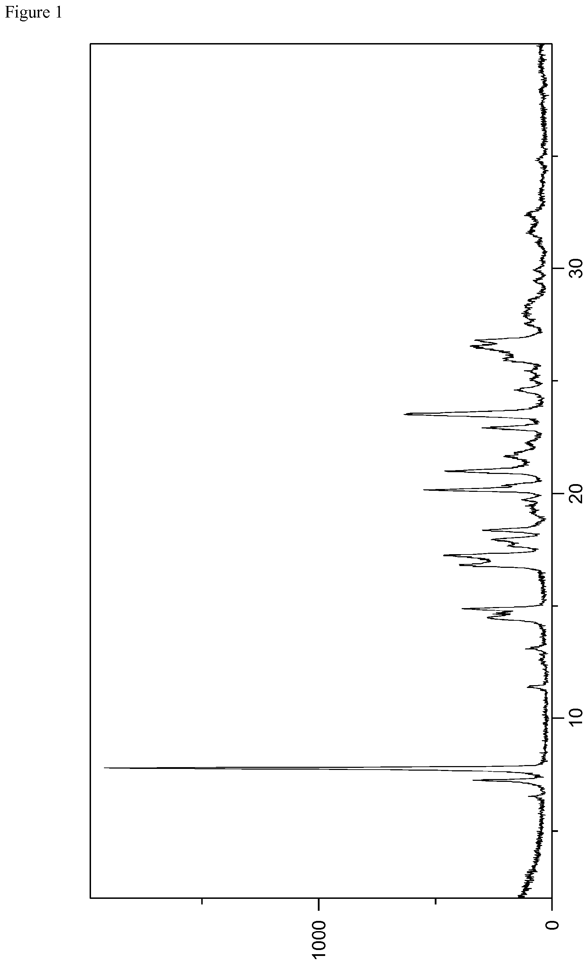

The present invention also relates to a salt comprising bictegravir and 1,5-naphtalenedisulfonic acid characterized by having a PXRD essentially the same as shown in Figure 1 of the present invention, when measured at a temperature in the range of from 20 to 30 °C with Cu-Kalpha_{1,2} radiation having a wavelength of 0.15419 nm.

Moreover, the present invention relates to a salt comprising bictegravir and 1,5-naphtalenedisulfonic acid characterized by having an FTIR spectrum comprising peaks at wavenumbers of:
(3081 ± 4) cm⁻¹, (2982 ± 4) cm⁻¹ and (1640 ± 4) cm⁻¹ or;
(3230 ± 4) cm⁻¹, (3081 ± 4) cm⁻¹, (2982 ± 4) cm⁻¹ and (1640 ± 4) cm⁻¹; or
(3230 ± 4) cm⁻¹, (3081 ± 4) cm⁻¹, (2982 ± 4) cm⁻¹, (1640 ± 4) cm⁻¹ and (1030 ± 4) cm⁻¹; or
(3230 ± 4) cm⁻¹, (3081 ± 4) cm⁻¹, (2982 ± 4) cm⁻¹, (1640 ± 4) cm⁻¹, (1442 ± 4) cm⁻¹ and (1030 ± 4) cm⁻¹; or
(3230 ± 4) cm⁻¹, (3081 ± 4) cm⁻¹, (2982 ± 4) cm⁻¹, (1640 ± 4) cm⁻¹, (1442 ± 4) cm⁻¹, (1304 ± 4) cm⁻¹ and (1030 ± 4) cm⁻¹; or
(3230 ± 4) cm⁻¹, (3081 ± 4) cm⁻¹, (2982 ± 4) cm⁻¹, (1640 ± 4) cm⁻¹, (1487 ± 4) cm⁻¹, (1442 ± 4) cm⁻¹, (1304 ± 4) cm⁻¹ and (1030 ± 4) cm⁻¹; or
(3230 ± 4) cm⁻¹, (3081 ± 4) cm⁻¹, (2982 ± 4) cm⁻¹, (1640 ± 4) cm⁻¹, (1487 ± 4) cm⁻¹, (1442 ± 4) cm⁻¹, (1304 ± 4) cm⁻¹, (1069 ± 4) cm⁻¹ and (1030 ± 4) cm⁻¹,
when measured at a temperature in the range of from 20 to 30 °C with a diamond ATR cell.

In addition, the present invention relates to a salt comprising bictegravir and 1,5-naphtalenedisulfonic acid characterized by having an FTIR spectrum comprising peaks at wavenumbers of:
(3081 ± 2) cm⁻¹, (2982 ± 2) cm⁻¹ and (1640 ± 2) cm⁻¹ or;
(3230 ± 2) cm⁻¹, (3081 ± 2) cm⁻¹, (2982 ± 2) cm⁻¹ and (1640 ± 2) cm⁻¹; or
(3230 ± 2) cm⁻¹, (3081 ± 2) cm⁻¹, (2982 ± 2) cm⁻¹, (1640 ± 2) cm⁻¹ and (1030 ± 2) cm⁻¹; or (3230 ± 2) cm⁻¹, (3081 ± 2) cm⁻¹, (2982 ± 2) cm⁻¹, (1640 ± 2) cm⁻¹, (1442 ± 2) cm⁻¹ and (1030 ± 2) cm⁻¹; or
(3230 ± 2) cm⁻¹, (3081 ± 2) cm⁻¹, (2982 ± 2) cm⁻¹, (1640 ± 2) cm⁻¹, (1442 ± 2) cm⁻¹, (1304 ± 2) cm⁻¹ and (1030 ± 2) cm⁻¹; or
(3230 ± 2) cm⁻¹, (3081 ± 2) cm⁻¹, (2982 ± 2) cm⁻¹, (1640 ± 2) cm⁻¹, (1487 ± 2) cm⁻¹, (1442 ± 2) cm⁻¹, (1304 ± 2) cm⁻¹ and (1030 ± 2) cm⁻¹; or
(3230 ± 2) cm⁻¹, (3081 ± 2) cm⁻¹, (2982 ± 2) cm⁻¹, (1640 ± 2) cm⁻¹, (1487 ± 2) cm⁻¹, (1442 ± 2) cm⁻¹, (1304 ± 2) cm⁻¹, (1069 ± 2) cm⁻¹ and (1030 ± 2) cm⁻¹,
when measured at a temperature in the range of from 20 to 30 °C with a diamond ATR cell.

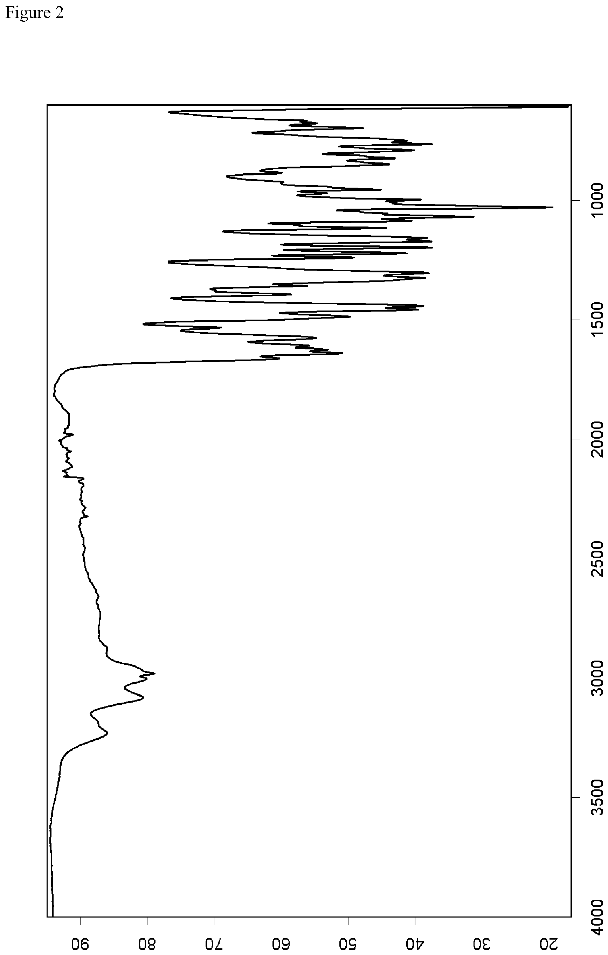

Alternatively, the present invention relates to a salt comprising bictegravir and 1,5-naphtalenedisulfonic acid characterized by having an FTIR spectrum essentially the same as shown in Figure 2 of the present invention, when measured at a temperature in the range of from 20 to 30 °C with a diamond ATR cell.

Furthermore, the present invention relates to a salt comprising bictegravir and 1,5-naphtalenedisulfonic acid characterized by having a DSC curve comprising an endothermic peak, preferably a single endothermic peak, having an onset temperature of (211 ± 5)°C, preferably of (211 ± 3)°C, even more preferably of (211 ± 2)°C and most preferably of (211 ± 1)°C, when measured at a heating rate of 10 K/min.

The present invention also relates to a salt comprising bictegravir and 1,5-naphtalenedisulfonic acid characterized by having a DSC curve comprising an endothermic peak, preferably a single endothermic peak, having a peak maximum temperature of (219 ± 5)°C, preferably of (219 ± 3)°C, even more preferably of (219 ± 2)°C and most preferably of (219 ± 1)°C, when measured a heating rate of 10 K/min.

Moreover, the invention relates to a salt comprising bictegravir and 1,5-naphtalenedisulfonic acid characterized by having a DSC curve showing an endothermic peak, preferably a single endothermic peak with an enthalpy of (102 ± 5) J/g, preferably of (102 ± 3) J/g, even more preferably of (102 ± 2) J/g and most preferably of (102 ± 1) J/g, when measured at a heating rate of 10 K/min.

Alternatively, the invention relates to a salt comprising bictegravir and 1,5-naphtalenedisulfonic acid characterized by having a melting point onset temperature of (211 ± 5)°C, preferably of (211 ± 3)°C, even more preferably of (211 ± 2)°C and most preferably of (211 ± 1)°C, when measured with DSC at a heating rate of 10 K/min.

In addition, the invention relates to a salt comprising bictegravir and 1,5-naphtalenedisulfonic acid characterized by having a melting point peak maximum temperature of (219 ± 5)°C, preferably of (219 ± 3)°C, even more preferably of (219 ± 2)°C and most preferably of (219 ± 1)°C, when measured with DSC at a heating rate of 10 K/min.

The present invention also relates to a salt comprising bictegravir and 1,5-naphtalenedisulfonic acid characterized by having a TGA curve showing a mass loss of not more than 2.0 w-%, preferably of not more than 1.5 w-%, even more preferably of not more than 1.0 w-%, e.g. a mass loss of about 1.0 w-% based on the weight of the salt, when heated from 25 to 170 °C at a heating rate of 10 K/min.

Furthermore, the present invention relates to a salt comprising bictegravir and 1,5-naphtalenedisulfonic acid characterized by showing a mass change of not more than 2.0 w-%, preferably of not more than 1.5 w-%, more preferably of not more than 1.0 w-% based on the weight of the salt, when measured with GMS in the sorption cycle at a relative humidity in the range of from 0 to 90% and a temperature of (25.0 ± 0.1)°C.

Moreover, the present invention relates to a salt comprising bictegravir and 1,5-naphtalenedisulfonic acid characterized by having a water content of not more than 2.0 w-%, preferably of not more than 1.5 w-% and most preferably of not more than 1.0 w-% based on the weight of the salt, as determined by Karl-Fischer titration at a relative humidity in the range of from 0 to 90% and a temperature of (25.0 ± 0.1)°C.

The present invention also relates to a salt comprising bictegravir and 1,5-naphtalenedisulfonic acid characterized as being an anhydrous salt.

Moreover, the present invention relates to a salt comprising bictegravir and 1,5-naphtalenedisulfonic acid characterized as being a non-solvated salt.

The present invention also relates to a salt comprising bictegravir and 1,5-naphtalenedisulfonic acid characterized by a spherical particle shape. Preferably, the diameter of the spheres is in the range of from 1 to 100 micrometer, more preferably of from 5 to 50 micrometer, even more preferably of from 10 to 30 micrometer.

In another aspect, the invention relates to a process for preparing the salt comprising bictegravir and 1,5-naphtalenedisulfonic acid as defined in any one of the above described embodiments comprising:
(a) reacting bictegravir and 1,5-naphtalenedisulfonic acid in the presence of a suitable solvent;
(b) allowing for the crystallization of the salt;
(c) separating at least a part of the crystals obtained in step (b);
(d) optionally washing the crystals obtained in step (c); and
(e) drying the crystals obtained in step (c) or (d).

Bictegravir may be prepared according to the teaching of WO 2014/100323 A1, in particular according to the teaching of Example 42 therein. 1,5-Naphtalenedisulfonic acid is a commercially available acid (e.g. 1,5-naphatalenedisulfonic acid tetrahydrate from Sigma-Aldrich®). In the first step (a) of the process bictegravir is reacted with 1,5-naphtalenedisulfonic acid in the presence of a suitable solvent, wherein the suitable solvent is selected from the group consisting of alcohols, ketones and cyclic ethers. Preferably, the alcohol is a C₁-C₃ alcohol e.g. selected from the group consisting of methanol, ethanol, 1-propanol and 2-propanol, the ketone is e.g. selected from acetone and ethylmehyl ketone and the cyclic ether is e.g. selected from tetrahydrofuran and 1,4-dioxane. More preferably, the alcohol is methanol, the ketone is acetone and the cyclic ether is tetrahydrofuran. The reaction may also be carried out in a solvent mixture comprising any one of the before listed solvents e.g. in a solvent mixture comprising methanol and tetrahydrofuran. Most preferably, the solvent used is acetone.

The molar ratio of bictegravir and 1,5-naphtalenedisulfonic acid applied in step (a) is in the range of from 1.0: 0.5-1.5, preferably of from 1.0: 0.6-0.9 and most preferably of from 1.0: 0.7-0.8 such as 1.00: 0.73, 1.00: 0.74, 1.00: 0.75, 1.00: 0.76 or 1.00: 0.77.

The bictegravir (free compound) concentration in relation to the applied solvent in step (a) is in the range of from 30 - 80 g/L, preferably of from 40 - 70 g/L and most preferably of from 50 - 60 g/L such as 54 g/L, 55 g/L or 56 g/L.

The reaction may be carried out at room temperature or at elevated temperature e.g. between room temperature and reflux temperature. Preferably, the reaction is accomplished at room temperature.

Once the reaction is complete the obtained salt is allowed to crystallize. Usually crystallization occurs spontaneously upon keeping the reaction mixture at room temperature, preferably under stirring. The obrained suspension may be further stirred until plentiful crystallization has occurred e.g. for a period in the range of from 0.5 to 24 hours, preferably of from 0.5 to 12 hours and most preferably of from 0.5 to 2 hours.

In the next step (c), at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

In an optional step (d), the isolated crystals may be washed with a suitable solvent, for example a solvent selected from the group consisting of alcohols, ketones and cyclic ethers. Preferably, the alcohol is a C₁-C₃ alcohol e.g. selected from the group consisting of methanol, ethanol, 1-propanol and 2-propanol, the ketone is e.g. selected from acetone and ethylmehyl ketone and the cyclic ether is e.g.selected from tetrahydrofuran and 1,4-dioxane. More preferably, the alcohol is methanol, the ketone is acetone and the cyclic ether is tetrahydrofuran. The washing step may also be carried out using a solvent mixture comprising any one of the before listed solvents e.g. in a solvent mixture comprising methanol and tetrahydrofuran. Most preferably, the solvent used is acetone.

Finally, the obtained crystals are dried, wherein drying may be performed at a temperature in the range of from about 20 to 100 °C, preferably of from about 20 to 60 °C , even more preferably of from about 20 to 40 °C and most preferably drying is performed at room temperature. Drying may be performed at ambient pressure and/or under reduced pressure. Preferably, drying is performed at a pressure of about 100 mbar or less, more preferably of about 50 mbar or less for example a vacuum of about 30 mbar or less. Drying may be performed for a period in the range of from about 1 to 24 hours, preferably from about 1 to 12 hours and most preferably from about 2 to 6 hours.

In another aspect, the present invention also relates to a salt comprising bictegravir and hydrochloric acid. In particular, the present invention relates to a salt comprising bictegravir and hydrochloric acid characterized by the chemical structure according to Formula (III) 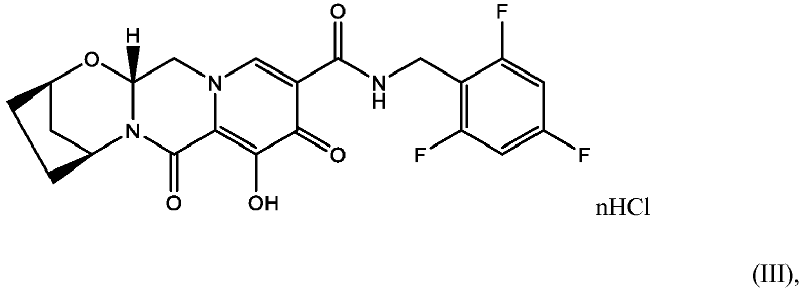 wherein n is in the range of from 0.8 to 1.2, preferably in the range of from 0.9 to 1.1, even more preferably in the range of from 0.95 to 1.05 and most preferably n is about 1.0. For example, the bictegravir salt of the present invention is a bictegravir mono-hydrochloride salt e.g. a salt having a molar ratio of bictegravir and hydrochloric acid in the range of from 1.0: 0.8 to 1.2, preferably in the range of from 1.0: 0.9 to 1.1, even more preferably in the range of from 1.00: 0.95 to 1.05 and most preferably the molar ratio is about 1.0 to 1.0.

In a particular preferred embodiment, the present invention relates to a salt comprising bictegravir and hydrochloric acid as defined in any one of the above described embodiments characterized in that the salt is crystalline.

The bictegravir hydrochloride salt of the present invention as defined in any one of the above described embodiments may be characterized by analytical methods well known in the field of the pharmaceutical industry for characterizing solids. Such methods comprise but are not limited to powder X-ray diffraction, Fourier transform infrared spectroscopy, differential scanning calorimetry, thermogravimetric analysis and gravimetric moisture sorption. It may be characterized by one of the aforementioned analytical methods or by combining two or more of them. In particular, the bictegravir hydrochloride salt of the present invention may be characterized by any one of the following embodiments or by combining two or more of the following embodiments.

The present invention relates to a salt comprising bictegravir and hydrochloric acid characterized by having a PXRD comprising reflections at 2-Theta angles of:
(8.9 ± 0.2)°, (16.5 ± 0.2)° and (18.1 ± 0.2); or
(7.6 ± 0.2)°, (8.9 ± 0.2)°, (16.5 ± 0.2)° and (18.1 ± 0.2); or
(7.6 ± 0.2)°, (8.9 ± 0.2)°, (16.5 ± 0.2)°, (18.1 ± 0.2)° and (26.1 ± 0.2)°; or
(7.6 ± 0.2)°, (8.9 ± 0.2)°, (15.2 ± 0.2)°, (16.5 ± 0.2)°, (18.1 ± 0.2)° and (26.1 ± 0.2)°; or
(6.3 ± 0.2)°, (7.6 ± 0.2)°, (8.9 ± 0.2)°, (15.2 ± 0.2)°, (16.5 ± 0.2)°, (18.1 ± 0.2)° and (26.1 ± 0.2)°; or
(6.3 ± 0.2)°, (7.6 ± 0.2)°, (8.9 ± 0.2)°, (15.2 ± 0.2)°, (16.5 ± 0.2)°, (17.5 ± 0.2)°, (18.1 ± 0.2)° and (26.1 ± 0.2)°; or
(6.3 ± 0.2)°, (7.6 ± 0.2)°, (8.9 ± 0.2)°, (15.2 ± 0.2)°, (16.5 ± 0.2)°, (17.1 ± 0.2)°, (17.5 ± 0.2)°, (18.1 ± 0.2)° and (26.1 ± 0.2)°; or
(6.3 ± 0.2)°, (7.6 ± 0.2)°, (8.9 ± 0.2)°, (13.5 ± 0.2)°, (15.2 ± 0.2)°, (16.5 ± 0.2)°, (17.1 ± 0.2)°, (17.5 ± 0.2)°, (18.1 ± 0.2)° and (26.1 ± 0.2)°,
when measured at a temperature in the range of from 20 to 30 °C with Cu-Kalpha_{1,2} radiation having a wavelength of 0.15419 nm.

Alternatively, the present invention relates to a salt comprising bictegravir and hydrochloric acid characterized by having a PXRD comprising reflections at 2-Theta angles of:
(8.9 ± 0.1)°, (16.5 ± 0.1)° and (18.1 ± 0.1); or
(7.6 ± 0.1)°, (8.9 ± 0.1)°, (16.5 ± 0.1)° and (18.1 ± 0.1); or
(7.6 ± 0.1)°, (8.9 ± 0.1)°, (16.5 ± 0.1)°, (18.1 ± 0.1)° and (26.1 ± 0.1)°; or
(7.6 ± 0.1)°, (8.9 ± 0.1)°, (15.2 ± 0.1)°, (16.5 ± 0.1)°, (18.1 ± 0.1)° and (26.1 ± 0.1)°; or
(6.3 ± 0.1)°, (7.6 ± 0.1)°, (8.9 ± 0.1)°, (15.2 ± 0.1)°, (16.5 ± 0.1)°, (18.1 ± 0.1)° and (26.1 ± 0.1)°; or
(6.3 ± 0.1)°, (7.6 ± 0.1)°, (8.9 ± 0.1)°, (15.2 ± 0.1)°, (16.5 ± 0.1)°, (17.5 ± 0.1)°, (18.1 ± 0.1)° and (26.1 ± 0.1)°; or
(6.3 ± 0.1)°, (7.6 ± 0.1)°, (8.9 ± 0.1)°, (15.2 ± 0.1)°, (16.5 ± 0.1)°, (17.1 ± 0.1)°, (17.5 ± 0.1)°, (18.1 ± 0.1)° and (26.1 ± 0.1)°; or
(6.3 ± 0.1)°, (7.6 ± 0.1)°, (8.9 ± 0.1)°, (13.5 ± 0.1)°, (15.2 ± 0.1)°, (16.5 ± 0.1)°, (17.1 ± 0.1)°, (17.5 ± 0.1)°, (18.1 ± 0.1)° and (26.1 ± 0.1)°,
when measured at a temperature in the range of from 20 to 30 °C with Cu-Kalpha_{1,2} radiation having a wavelength of 0.15419 nm.

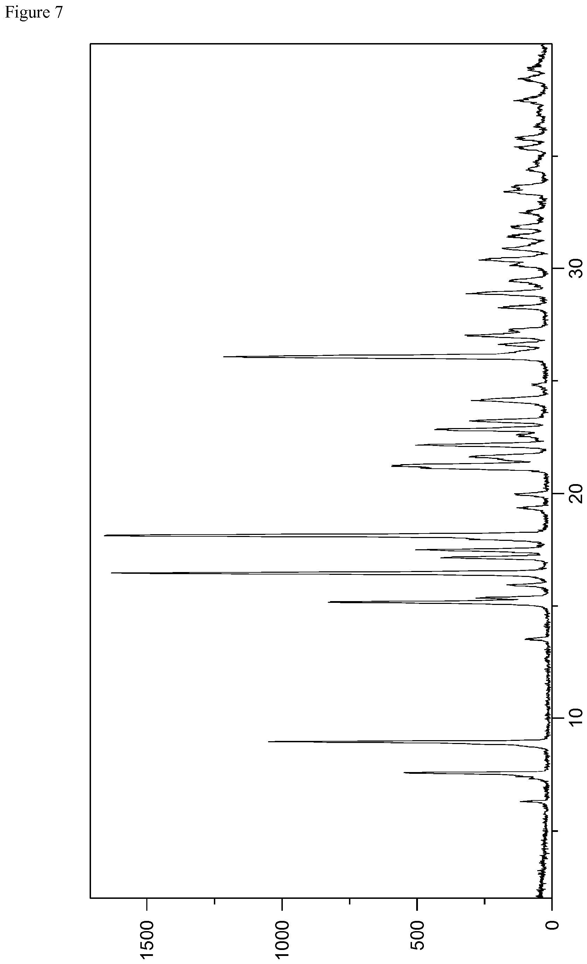

The present invention also relates to a salt comprising bictegravir and hydrochloric acid characterized by having a PXRD essentially the same as shown in Figure 7 of the present invention, when measured at a temperature in the range of from 20 to 30 °C with Cu-Kalpha_{1,2} radiation having a wavelength of 0.15419 nm.

Moreover, the present invention relates to a salt comprising bictegravir and hydrochloric acid characterized by having an FTIR spectrum comprising peaks at wavenumbers of:
(2953 ± 4) cm⁻¹, (2863 ± 4) cm⁻¹ and (1639 ± 4) cm⁻¹ or;
(2953 ± 4) cm⁻¹, (2863 ± 4) cm⁻¹, (1639 ± 4) cm⁻¹ and (1067 ± 4) cm⁻¹; or
(2953 ± 4) cm⁻¹, (2863 ± 4) cm⁻¹, (1639 ± 4) cm⁻¹, (1067 ± 4) cm⁻¹ and (848 ± 4) cm⁻¹; or
(2953 ± 4) cm⁻¹, (2863 ± 4) cm⁻¹, (1639 ± 4) cm⁻¹, (1438 ± 4) cm⁻¹, (1067 ± 4) cm⁻¹ and (848 ± 4) cm⁻¹; or
(2953 ± 4) cm⁻¹, (2863 ± 4) cm⁻¹, (1639 ± 4) cm⁻¹, (1540 ± 4) cm⁻¹, (1438 ± 4) cm⁻¹, (1067 ± 4) cm⁻¹ and (848 ± 4) cm⁻¹; or
(2953 ± 4) cm⁻¹, (2863 ± 4) cm⁻¹, (1639 ± 4) cm⁻¹, (1540 ± 4) cm⁻¹, (1438 ± 4) cm⁻¹, (1333 ± 2) cm⁻¹, (1067 ± 4) cm⁻¹ and (848 ± 4) cm⁻¹; or
(2953 ± 4) cm⁻¹, (2863 ± 4) cm⁻¹, (1639 ± 4) cm⁻¹, (1540 ± 4) cm⁻¹, (1438 ± 4) cm⁻¹, (1333 ± 4) cm⁻¹, (1197 ± 4) cm⁻¹, (1067 ± 4) cm⁻¹ and (848 ± 4) cm⁻¹,
when measured at a temperature in the range of from 20 to 30 °C with a diamond ATR cell.

In addition, the present invention relates to a salt comprising bictegravir and hydrochloric acid characterized by having an FTIR spectrum comprising peaks at wavenumbers of:
(2953 ± 2) cm⁻¹, (2863 ± 2) cm⁻¹ and (1639 ± 2) cm⁻¹ or;
(2953 ± 2) cm⁻¹, (2863 ± 2) cm⁻¹, (1639 ± 2) cm⁻¹ and (1067 ± 2) cm⁻¹; or
(2953 ± 2) cm⁻¹, (2863 ± 2) cm⁻¹, (1639 ± 2) cm⁻¹, (1067 ± 2) cm⁻¹ and (848 ± 2) cm⁻¹; or
(2953 ± 2) cm⁻¹, (2863 ± 2) cm⁻¹, (1639 ± 2) cm⁻¹, (1438 ± 2) cm⁻¹, (1067 ± 2) cm⁻¹ and (848 ± 2) cm⁻¹; or
(2953 ± 2) cm⁻¹, (2863 ± 2) cm⁻¹, (1639 ± 2) cm⁻¹, (1540 ± 2) cm⁻¹, (1438 ± 2) cm⁻¹, (1067 ± 2) cm⁻¹ and (848 ± 2) cm⁻¹; or
(2953 ± 2) cm⁻¹, (2863 ± 2) cm⁻¹, (1639 ± 2) cm⁻¹, (1540 ± 2) cm⁻¹, (1438 ± 2) cm⁻¹, (1333 ± 2) cm⁻¹, (1067 ± 2) cm⁻¹ and (848 ± 2) cm⁻¹; or
(2953 ± 2) cm⁻¹, (2863 ± 2) cm⁻¹, (1639 ± 2) cm⁻¹, (1540 ± 2) cm⁻¹, (1438 ± 2) cm⁻¹, (1333 ± 2) cm⁻¹, (1197 ± 2) cm⁻¹, (1067 ± 2) cm⁻¹ and (848 ± 2) cm⁻¹,
when measured at a temperature in the range of from 20 to 30 °C with a diamond ATR cell.

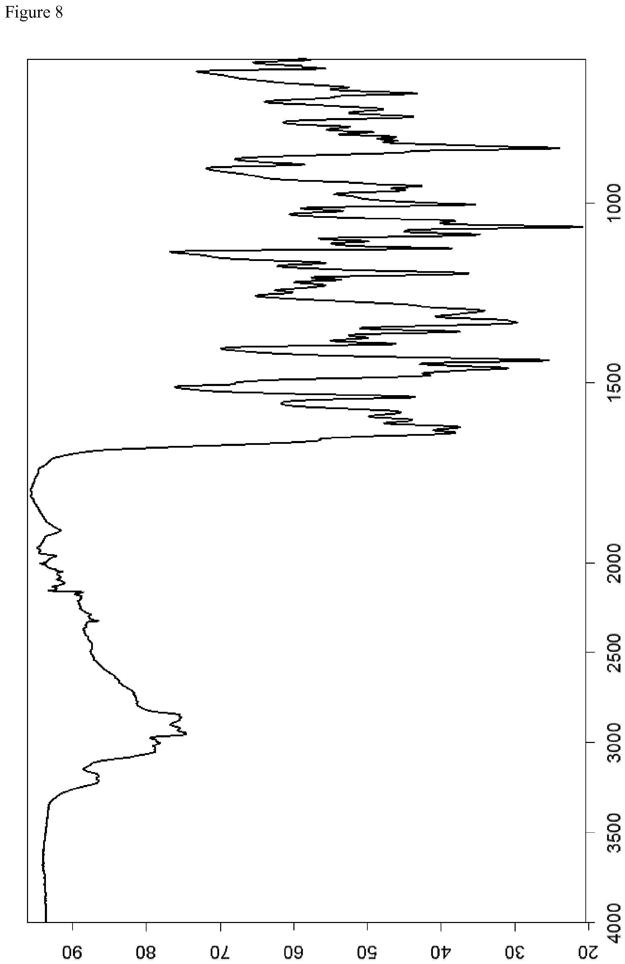

Alternatively, the present invention relates to a salt comprising bictegravir and hydrochloric acid characterized by having an FTIR spectrum essentially the same as shown in Figure 8 of the present invention, when measured at a temperature in the range of from 20 to 30 °C with a diamond ATR cell.

Furthermore, the present invention relates to a salt comprising bictegravir and hydrochloric acid characterized by having a DSC curve comprising an endothermic peak, preferably a first endothermic peak, having an onset temperature of (145 ± 5)°C, preferably of (145 ± 3)°C, even more preferably of (145 ± 1)°C, when measured at a heating rate of 10 K/min.

The present invention also relates to a salt comprising bictegravir and hydrochloric acid characterized by having a DSC curve comprising an endothermic peak, preferably a first endothermic peak, having a peak maximum temperature of (163 ± 5)°C, preferably of (163 ± 3)°C, even more preferably of (163 ± 1)°C, when measured a heating rate of 10 K/min.

Moreover, the invention relates to a salt comprising bictegravir and hydrochloric acid characterized by having a DSC curve showing an endothermic peak, preferably a first endothermic peak with an enthalpy of (115 ± 5) J/g, preferably of (115 ± 3) J/g, even more preferably of (115 ± 1) J/g, when measured at a heating rate of 10 K/min.

Furthermore, the present invention relates to a salt comprising bictegravir and hydrochloric acid characterized by having a DSC curve comprising an endothermic peak, preferably a second endothermic peak, having an onset temperature of (228 ± 5)°C, preferably of (228 ± 3)°C, even more preferably of (228 ± 1)°C, when measured at a heating rate of 10 K/min.

The present invention also relates to a salt comprising bictegravir and hydrochloric acid characterized by having a DSC curve comprising an endothermic peak, preferably a second endothermic peak, having a peak maximum temperature of (231 ± 5)°C, preferably of (231 ± 3)°C, even more preferably of (231 ± 1)°C, when measured a heating rate of 10 K/min.

Moreover, the invention relates to a salt comprising bictegravir and hydrochloric acid characterized by having a DSC curve showing an endothermic peak, preferably a second endothermic peak with an enthalpy of (56 ± 5) J/g, preferably of (56± 3) J/g, even more preferably of (56 ± 1) J/g, when measured at a heating rate of 10 K/min.

The present invention also relates to a salt comprising bictegravir and hydrochloric acid characterized by having a TGA curve showing a mass loss, preferably a first mass loss of not more than 0.5 w-% based on the weight of the salt, when heated from 25 to 100 °C at a rate of 10 K/min.

Furthermore, the present invention also relates to a salt comprising bictegravir and hydrochloric acid characterized by having a TGA curve showing a mass loss, preferably a second mass loss, of not more than 6.5 w-% based on the weight of the salt, when heated from 100 to 215 °C at a rate of 10 K/min.

Furthermore, the present invention relates to a salt comprising bictegravir and hydrochloric acid characterized by showing a mass change of not more than 2.0 w-%, preferably of not more than 1.5 w-%, most preferably of not more than 1.0 w-% based on the weight of the salt, when measured with GMS in the sorption cycle at a relative humidity in the range of from 0 to 90% and a temperature of (25.0 ± 0.1)°C.

Moreover, the present invention relates to a salt comprising bictegravir and hydrochloric acid characterized by having a water content of not more than 2.0 w-%, preferably of not more than 1.5 w-% and most preferably of not more than 1.0 w-% based on the weight of the salt, as determined by Karl-Fischer titration at a relative humidity in the range of from 0 to 90% and a temperature of (25.0 ± 0.1)°C.

The present invention also relates to a salt comprising bictegravir and hydrochloric acid characterized as being an anhydrous salt.

Moreover, the present invention relates to a salt comprising bictegravir and hydrochloric acid characterized as being a non-solvated salt.

The present invention also relates to a salt comprising bictegravir and hydrochloric acid characterized by a lath-shaped crystal morphology.

In another aspect, the invention relates to a process for preparing a salt comprising bictegravir and hydrochloric acid as defined in any one of the above described embodiments comprising:
(a) reacting bictegravir and hydrochloric acid in the presence of a suitable solvent;
(b) allowing for the crystallization of the salt;
(c) separating at least a part of the crystals obtained in step (b);
(d) optionally washing the crystals obtained in step (c); and
(e) drying the crystals obtained in step (c) or (d).

Bictegravir may be prepared according to the teaching of WO 2014/100323 A1, in particular according to the teaching of Example 42 therein. Hydrochloric acid is preferably applied as aqueous solution, most preferably concentrated hydrochloric acid (assay 36-37%) is used for the described process. In the first step (a) of the process bictegravir is reacted with hydrochloric acid in the presence of a suitable solvent, wherein the suitable solvent is selected from ketones such as acetone and ethylmethyl ketone or water. Most preferably, the solvent used is acetone.

The molar ratio of bictegravir and hydrochloric acid applied in step (a) is in the range of from 1.0: 2.0 - 15.0, preferably of from 1.0: 5.0 - 12.0, and most preferably the molar ratio applied is about 1.0: 10.0

The bictegravir (free compound) concentration in relation to the solvent applied in step (a) is in the range of from 60- 100 g/L, preferably of from 70 - 90 g/L and most preferably of from 80 - 90 g/L such as 83 g/L, 84 g/L, 85 g/L, 86 g/L or 87 g/L.

The reaction may be carried out at room temperature or at elevated temperature e.g. between room temperature and reflux temperature. Preferably, the reaction is accomplished at room temperature.

Once the reaction is complete the obtained salt is allowed to crystallize. Usually crystallization occurs spontaneously upon keeping the reaction mixture at room temperature, preferably under stirring. The obtained suspension may be further stirred until plentiful crystallization has occurred e.g. for a period in the range of from 0.5 to 24 hours, preferably of from 0.5 to 12 hours and most preferably of from 0.5 to 2 hours. In order to improve the yield, the obtained suspension may be cooled e.g. to a temperature in the range of from about -5 - 15 °C, such as about 0 °C.

In the next step (c), at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

In an optional step (d), the isolated crystals may be washed with a suitable solvent, for example a solvent selected from ketones such as acetone and ethylmethyl ketone or water. Most preferably, the solvent used is acetone.

Finally, the obtained crystals are dried, wherein drying may be performed at a temperature in the range of from about 20 to 100 °C, preferably of from about 20 to 60 °C , even more preferably of from about 20 to 40 °C and most preferably drying is performed at room temperature. Drying may be performed at ambient pressure and/or under reduced pressure. Preferably, drying is performed at a pressure of about 100 mbar or less, more preferably of about 50 mbar or less for example a vacuum of about 30 mbar or less. Drying may be performed for a period in the range of from about 1 to 24 hours, preferably from about 1 to 12 hours and most preferably from about 2 to 6 hours.

In a further aspect the present invention relates to the use of bictegravir *hemi*-napadisylate or bictegravir *mono*-hydrochloride of the present invention as defined above for the preparation of a pharmaceutical composition.

In a further aspect, the present invention relates to a pharmaceutical composition comprising bictegravir *hemi*-napadisylate or bictegravir *mono*-hydrochloride of the present invention as defined above, preferably in an effective and/or predetermined amount, and at least one pharmaceutically acceptable excipient. Most preferably, the pharmaceutical composition of the present invention is an oral solid dosage form, such as a tablet or a capsule. Preferably, the pharmaceutical composition of the present invention is a tablet, most preferably a film-coated tablet. In one embodiment, the tablet is film-coated with a coating material containing polyvinyl alcohol (e.g. partially hydrolyzed), iron oxide, talc, and titanium dioxide.

The at least one pharmaceutically acceptable excipient, which is comprised in the pharmaceutical composition of the present invention, is preferably selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof. More preferably, the at least one pharmaceutically acceptable excipient is selected from the group consisting of fillers, diluents, lubricants, disintegrants and coating materials. In one embodiment all of these pharmaceutically acceptable excipients are comprised by the pharmaceutical composition of the present invention.

In a preferred embodiment, the at least one pharmaceutically acceptable excipient is selected from the group consisting of microcrystalline cellulose, croscarmellose sodium and magnesium stearate. In a preferred embodiment, all of these pharmaceutically acceptable excipients are comprised by the pharmaceutical composition of the present invention.

Preferably, the present invention relates to a pharmaceutical composition as describe above, wherein the predetermined and/or effective amount of bictegravir *hemi*-napadisylate or bictegravir *mono*-hydrochloride is selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg calculated as bictegravir. Most preferably, the invention relates to a pharmaceutical composition as describe above, wherein the predetermined and/or effective amount of bictegravir *hemi*-napadisylate or bictegravir *mono*-hydrochloride is 50 mg calculated as bictegravir.

In a further aspect, the present invention relates to a pharmaceutical composition comprising bictegravir *hemi*-napadisylate or bictegravir *mono*-hydrochloride of the present invention as defined above, preferably in an effective and/or predetermined amount, and at least one pharmaceutically acceptable excipient and optionally one or more additional active pharmaceutical ingredient(s). Most preferably, the pharmaceutical composition of the present invention is an oral solid dosage form, such as a tablet or a capsule. Preferably, the pharmaceutical composition of the present invention is a tablet, most preferably a film-coated tablet. In one embodiment, the tablet is film-coated with a coating material containing polyvinyl alcohol (e.g. partially hydrolyzed), iron oxide, talc, and titanium dioxide.

The at least one pharmaceutically acceptable excipient, which is comprised in the pharmaceutical composition of the present invention, is preferably selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof. More preferably, the at least one pharmaceutically acceptable excipient is selected from the group consisting of fillers, diluents, lubricants, disintegrants and coating materials. In one embodiment all of these pharmaceutically acceptable excipients are comprised by the pharmaceutical composition of the present invention.

In a preferred embodiment, the at least one pharmaceutically acceptable excipient is selected from the group consisting of microcrystalline cellulose, croscarmellose sodium and magnesium stearate. In a preferred embodiment, all of these pharmaceutically acceptable excipients are comprised by the pharmaceutical composition of the present invention.

Preferably, the present invention relates to a pharmaceutical composition as describe above, wherein the predetermined and/or effective amount of bictegravir *hemi*-napadisylate or bictegravir *mono*-hydrochloride is selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg calculated as bictegravir. Most preferably, the invention relates to a pharmaceutical composition as describe above, wherein the predetermined and/or effective amount of bictegravir *hemi*-napadisylate or bictegravir *mono*-hydrochloride is 50 mg calculated as bictegravir.

In another preferred embodiment, the one or more additional active pharmaceutical ingredient(s) is/are selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

In a particular preferred embodiment, the one or more additional active pharmaceutical ingredient(s) is/are selected from the group consisting of emtricitabine or a pharmaceutically acceptable salt thereof and tenofovir alafenamide or a pharmaceutically acceptable salt thereof, most preferably the one or more additional active pharmaceutical ingredients are emtricitabine and tenofovir alafenamide hemifumarate.

In certain embodiments the pharmaceutical composition comprises 50 mg bictegravir *hemi-*napadisylate or bictegravir *mono*-hydrochloride calculated as bictegravir, 25 mg tenofovir alafenamide or a pharmaceutically acceptable salt thereof calculated as tenofovir alafenamide, and 200 mg emtricitabine or a pharmaceutically acceptable salt thereof calculated as emtricitabine. For instance, in certain embodiments, the pharmaceutical composition comprises 66.0 mg bictegravir *hemi*-napadisylate or 54.1 mg bictegravir *mono*-hydrochloride, 28 mg tenofovir alafenamide hemifumarate and 200 mg emtricitabine. In a particular preferred embodiment, the pharmaceutical composition of the present invention is a bilayer tablet, comprising a first layer comprising bictegravir *hemi*-napadisylate or bictegravir *mono-*hydrochloride of the present invention as defined above and a second layer comprising tenofovir alafenamide or a pharmaceutically acceptable salt thereof and emtricitabine or a pharmaceutically acceptable salt thereof. Preferably, the invention relates to a bilayer tablet, comprising a first layer bictegravir *hemi*-napadisylate or bictegravir *mono*-hydrochloride of the present invention as defined above and a second layer comprising tenofovir alafenamide hemifumarate and emtricitabine.

Preferably, the present invention relates to a pharmaceutical composition as described above, wherein the pharmaceutical composition is to be administered once-daily.

In a further aspect, the present invention relates to the pharmaceutical composition as described above for use as a medicament.

In yet another aspect, the present invention relates to the pharmaceutical composition as described above for use in the treatment or prophylaxis of viral infections caused by DNA viruses, RNA viruses, herpesviruses (e.g. CMV, HSV 1, HSV 2, VZV), retroviruses, hepadnaviruses (e.g. HBV), papillomavirus, hantavirus, adenoviruses and HIV.

In a particular embodiment, the present invention relates to the pharmaceutical composition as described above for use in the treatment and/or prophylaxis of HIV-1 infections.

In another embodiment, the present invention is directed to a method of treating or prophylactically preventing HIV-1 infections by administering the pharmaceutical composition as described above to a patient in need of such a treatment and/or prophylaxis.

In another aspect the present invention relates to the pharmaceutical composition as described above intended for the treatment of HIV-1 infections in combination with one or more additional active pharmaceutical ingredient(s) selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc, ibalizumab and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

A treatment in combination with one or more additional active pharmaceutical ingredient(s) can mean the administration of a pharmaceutical dosage form comprising the bictegravir *hemi-*napadisylate or bictegravir *mono*-hydrochloride of the present invention as defined above and the one or more additional active pharmaceutical ingredient(s) in the same dosage form, for example as a fixed-dose combination.

Alternatively, a treatment in combination with one or more additional active pharmaceutical ingredient(s) can mean the administration of separate pharmaceutical dosage forms, one comprising the bictegravir *hemi*-napadisylate or bictegravir *mono*-hydrochloride of the present invention as defined above, and the other(s) comprising the one or more additional active pharmaceutical ingredient(s) in separate dosage form(s). Typically in such a combination treatment instructions are provided that the pharmaceutical dosage form comprising the bictegravir sodium form II of the present invention is to be administered in combination with said separate dosage form(s) for the effective treatment of a viral invention, such as HIV-1 infection.

The present invention is further illustrated by the following embodiments and combinations of embodiments resulting from the given dependencies and back-references:
1) A salt comprising bictegravir and 1,5-naphthalenedisulfonic acid.
2) The salt of embodiment 1 characterized by the chemical structure according to Formula 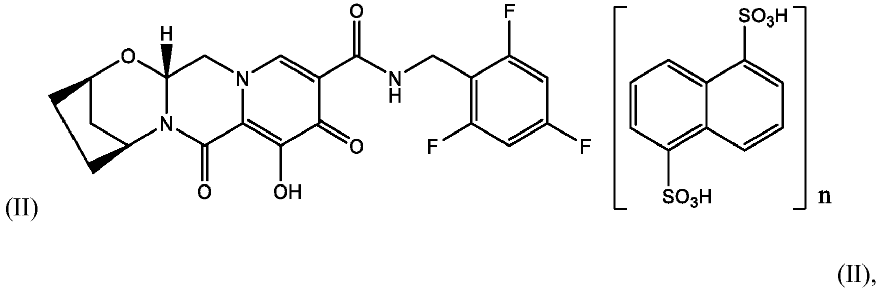 wherein n is in the range of from 0.4 to 0.6.
3) The salt as defined in embodiment 1 or 2 characterized in that the salt is crystalline.
4) The salt as defined in any one of the preceding embodiments characterized by having a powder X-ray diffractogram comprising reflections at 2-Theta angles of (7.2 ± 0.2)°, (7.8 ± 0.2)° and (23.5 ± 0.2)°, when measured at a temperature in the range of from 20 to 30 °C with Cu-Kalpha_{1,2} radiation having a wavelength of 0.15419 nm.
5) The salt of embodiment 4 characterized by having a powder X-ray diffractogram comprising additional reflections at 2-Theta angles of (20.1 ± 0.2)° and (21.0 ± 0.2)°, when measured at a temperature in the range of from 20 to 30 °C with Cu-Kalpha_{1,2} radiation having a wavelength of 0.15419 nm.
6) The salt as defined in any one of the preceding embodiments characterized by having a Fourier transform infrared spectrum comprising peaks at wavenumbers of (3230 ± 4) cm⁻¹, (3081 ± 4) cm⁻¹, (2982 ± 4) cm⁻¹, (1640 ± 4) cm⁻¹ and (1030 ± 4) cm⁻¹, when measured at a temperature in the range of from 20 to 30 °C with a diamond ATR cell.
7) The salt as defined in any one of the preceding embodiments characterized by having a melting point onset temperature of (211 ± 5)°C, when measured with DSC at a heating rate of 10 K/min.
8) The salt as defined in any one of the preceding embodiments characterized by having a thermogravimetric analysis curve showing a mass loss of not more than 2.0 weight-% based on the weight of the salt, when heated from 25 to 170 °C at a rate of 10 K/min.
9) The salt as defined in any one of the preceding embodiments characterized by showing a mass change of not more than 2.0 weight-% based on the weight of the salt, when measured with gravimetric moisture sorption in the sorption cycle at a relative humidity in the range of from 0 to 90% and a temperature of (25.0 ± 0.1)°C.
10) Use of the salt as defined in any one of the preceding embodiments for the preparation of a pharmaceutical composition.
11) A pharmaceutical composition comprising the salt as defined in any one of embodiments 1 to 9 and at least one pharmaceutically acceptable excipient.
12) The pharmaceutical compositon of embodiment 11, further comprising one or more additional active pharmaceutical ingredient(s).
13) The pharmaceutical composition of embodiment 12, wherein the one or more additional active pharmaceutical ingredients are selected from emtricitabine and tenofovir alafenamide hemifumarate.
14) The pharmaceutical composition as defined in any one of embodiments 11 to 13, wherein the pharmaceutical composition is an oral solid dosage form.
15) The pharmaceutical composition as defined in any one of embodiments 11 to 14 for use in the treatment and/or prophylaxis of HIV-1 infections.
16) A process for preparing the salt comprising bictegravir and 1,5-naphtalenedisulfonic acid as defined in any one of embodiments 1 to 9 comprising:
   (a) reacting bictegravir and 1,5-naphtalenedisulfonic acid in the presence of a suitable solvent;
   (b) allowing for the crystallization of the salt;
   (c) separating at least a part of the crystals obtained in step (b);and
   (d) drying the crystals obtained in step (c).
17) The process of embodiment 16, wherein the suitable solvent is selected from the group consisting of acetone, methanol and tetrahydrofuran or any mixture thereof.
18) A salt comprising bictegravir and hydrochloric acid.
19) The salt of embodiment 18 characterized by the chemical structure according to Formula (III) 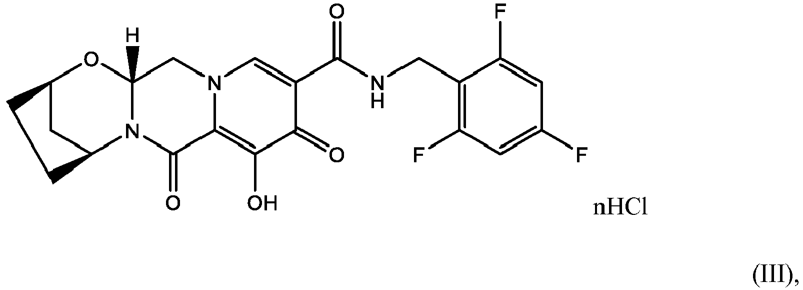 wherein n is in the range of from 0.8 to 1.2.
20) The salt as defined in embodiment 18 or 19 characterized in that the salt is crystalline.
21) The salt as defined in any one of embodiments 18 to 20 characterized by having a powder X-ray diffractogram comprising reflections at 2-Theta angles of (8.9 ± 0.2)°, (16.5 ± 0.2)° and (18.1 ± 0.2), when measured at a temperature in the range of from 20 to 30 °C with Cu-Kalpha_{1,2} radiation having a wavelength of 0.15419 nm.
22) The salt of embodiment 21 characterized by having a powder X-ray diffractogram comprising additional reflections at 2-Theta angles of (7.6 ± 0.2)° and (26.1 ± 0.2)°, when measured at a temperature in the range of from 20 to 30 °C with Cu-Kalpha_{1,2} radiation having a wavelength of 0.15419 nm.
23) The salt as defined in any one of embodiments 18 to 22 characterized by having a Fourier transform infrared spectrum comprising peaks at wavenumbers of (2953 ± 4) cm⁻¹, (2863 ± 4) cm⁻¹, (1639 ± 4) cm⁻¹, (1067 ± 4) cm⁻¹ and (848 ± 4) cm⁻¹, when measured at a temperature in the range of from 20 to 30 °C with a diamond ATR cell.
24) The salt as defined in any one of embodiments 18 to 23 characterized by having a DSC curve comprising and endothermic peak having an onset temperature of (145 ± 5)°C, when measured with DSC at a heating rate of 10 K/min.
25) The salt as defined in any one of embodiments 18 to 24 characterized by having a thermogravimetric analysis curve showing a mass loss of not more than 0.5 weight-% based on the weight of the salt, when heated from 25 to 100 °C at a rate of 10 K/min.
26) The salt as defined in any one of embodiments 18 to 25 characterized by showing a mass change of not more than 2.0 weight-% based on the weight of the salt, when measured with gravimetric moisture sorption in the sorption cycle at a relative humidity in the range of from 0 to 90% and a temperature of (25.0 ± 0.1)°C.
27) Use of the salt as defined in any one embodiments 18 to 26 for the preparation of a pharmaceutical composition.
28) A pharmaceutical composition comprising the salt as defined in any one of embodiments 18 to 26 and at least one pharmaceutically acceptable excipient.
29) The pharmaceutical compositon of embodiment 28, further comprising one or more additional active pharmaceutical ingredient(s).
30) The pharmaceutical composition of embodiment 29, wherein the one or more additional active pharmaceutical ingredients are selected from emtricitabine and tenofovir alafenamide hemifumarate.
31) The pharmaceutical composition as defined in any one of embodiments 28 to 30, wherein the pharmaceutical composition is an oral solid dosage form.
32) The pharmaceutical composition as defined in any one of embodiments 28 to 31 for use in the treatment and/or prophylaxis of HIV-1 infections.
33) A process for preparing the salt comprising bictegravir and hydrochloric acid as defined in any one of embodiments 18 to 26 comprising:
   (a) reacting bictegravir and hydrochloric acid in the presence of a suitable solvent;
   (b) allowing for the crystallization of the salt;
   (c) separating at least a part of the crystals obtained in step (b);and
   (d) drying the crystals obtained in step (c).
34) The process of embodiment 33, wherein the suitable solvent is selected from acetone, water or a mixture thereof.

### EXAMPLES

The following non-limiting examples are illustrative for the disclosure and are not to be construed as to be in any way limiting for the scope of the invention.

### Analytical Methods

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

FTIR spectra were recorded (obtained) on a MKII Golden Gate™ Single Reflection Diamond ATR cell with a Bruker Tensor 27 FTIR spectrometer with 4 cm⁻¹ resolution at a temperature in the range of from 20 to 30 °C. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of ± 4 cm^{- 1} preferably of ± 2 cm⁻¹.

DSC was performed on a Mettler Polymer DSC R instrument. The bictegravir *hemi-*napadisylate sample (4.98 mg) and the bictegravir *mono*-hydrochloride sample (3.78 mg) were each heated in a 40 microliter aluminium pan with a pierced aluminium lid from 25 to 250 °C at a rate of 10 K/min. Nitrogen (purge rate 50 mL/min) was used as purge gas.

TGA was performed on a Mettler TGA/DSC 1 instrument. The bictegravir *hemi*-napadisylate sample (10.08 mg) and the bictegravir *mono*-hydrochloride sample (6.16 mg) were each heated in a 100 microliter aluminum pan closed with an aluminum lid. The lid was automatically pierced at the beginning of the measurement. The samples were heated from 25 to 250 °C at a rate of 10 K/min. Nitrogen (purge rate 50 mL/min) was used as purge gas.

Gravimetric moisture sorption experiments conducted with bictegravir *hemi*-napadisylate and bictegravir *mono*-hydrochloride were recorded with an SPSx-1µ moisture sorption analyzer (ProUmid, Ulm). The measurement cycles for bictegravir *hemi*-napadisylate and bictegravir mono-hydrochloride were started at ambient RH of 25% and 30%, respectively. RH was then decreased to 5% in 5% steps, followed by a further decrease to 3% and to 0%. Afterwards RH was increased from 0% to approximately 90% in a sorption cycle and decreased to 0 % in a desorption cycle in 5% steps. Finally, RH was increased to 25% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C. At the end of the experiments the water contents of bictegravir *hemi*-napadisylate and bictegravir *mono*-hydrochloride were determined by coulometric Karl-Fischer titration on a Metrohm 831 KF Coulometer connected to a KF Thermoprep 832 oven, which was heated to 130 °C for the measurements.

Scanning electron microscopic images were recorded with a Hitachi TM3030Plus Tabletop Microscope using the detector in secondary electron/backscattering electron mixed mode at a voltage of 15 kV.

### Example 1: Preparation of bictegravir hemi-napadisylate

Bictegravir (33.3 g, 74.1 mmol, e.g. prepared according to Example 42 of WO 2014/100323 A1) was dissolved in acetone (600 mL) at 25 °C. Following addition of 1,5-naphtalenedisulfonic acid tetrahydrate (assay 97%, 19.9 g, 53.6 mmol) crystallization occurred and the obtained suspension was further stirred at 25 °C for 45 min. Then, the crystals were collected by filtration, washed twice with acetone (each 50 mL) and dried at room temperature under vacuum (30 mbar) for 2 hours to obtain crystalline bictegravir *hemi*-napadisylate (43.99 g).
Yield: 100% of theory

### Example 2: Solid-state characterization of bictegravir hemi-napadisylate

### Powder X-ray diffraction

A representative diffractogram of bictegravir *hemi*-napadisylate is displayed in Figure 1 herein. The corresponding reflection list is provided in Table 1 below.

**Table 1: PXRD reflections of bictegravir hemi-napadisylate in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 6.5 | 17.2 | 21.0 | 26.0 |
| 7.2 | 17.7 | 21.6 | 26.5 |
| 7.8 | 17.9 | 22.2 | 26.8 |
| 11.4 | 18.3 | 22.9 | 27.5 |
| 13.1 | 19.1 | 23.5 | 28.0 |
| 14.5 | 19.7 | 24.6 | 29.4 |
| 14.9 | 20.1 | 25.1 | 29.9 |
| 16.8 | 20.4 | 25.4 | |

### Fourier transform infrared spectroscopy

A representative FTIR spectrum of bictegravir *hemi*-napadisylate according to the present invention is displayed in Figure 2 and the corresponding peak list is provided in Table 2 below.

**Table 2: FTIR peak list of bictegravir hemi-napadisylate according to the present invention; a typical precision of the wavenumbers is in the range of ± 4 cm⁻¹, preferably of ± 2 cm⁻¹.**

| **Wavenumber [cm⁻¹]** | **Wavenumber [cm⁻¹]** | **Wavenumber [cm⁻¹]** |
|---|---|---|
| 3230 | 1394 | 1030 |
| 3081 | 1325 | 998 |
| 2982 | 1304 | 955 |
| 1640 | 1222 | 886 |
| 1625 | 1198 | 850 |
| 1577 | 1172 | 824 |
| 1534 | 1116 | 791 |
| 1487 | 1086 | 698 |
| 1442 | 1069 | 678 |

### Differential scanning calorimetry

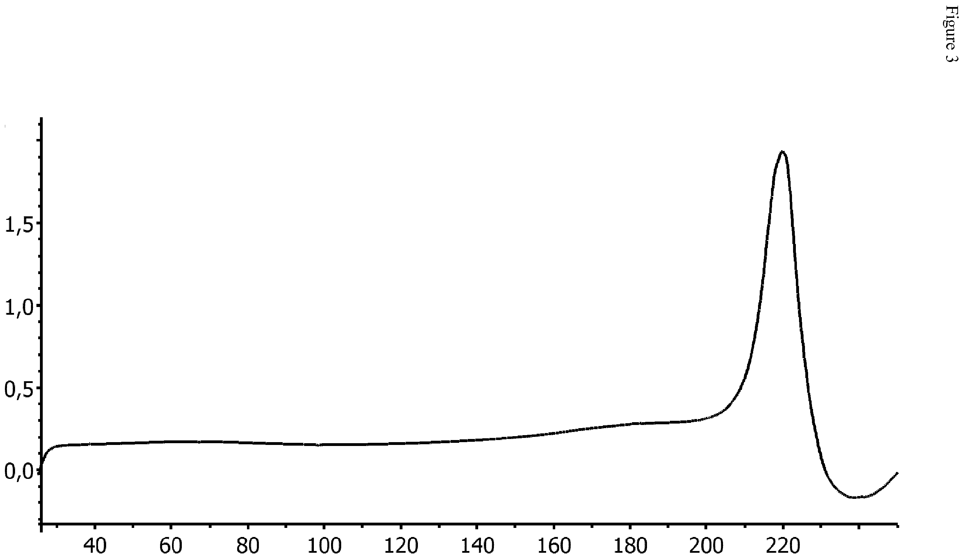

The DSC curve of bictegravir *hemi*-napadisylate shows an endothermic peak with an onset temperature of about 211 °C, a peak temperature of about 219 °C and an enthalpy of about 102 J/g, which is due to a concomitant melting and decomposition process (see also Figure 3 herein).

### Thermo gravimetric analysis

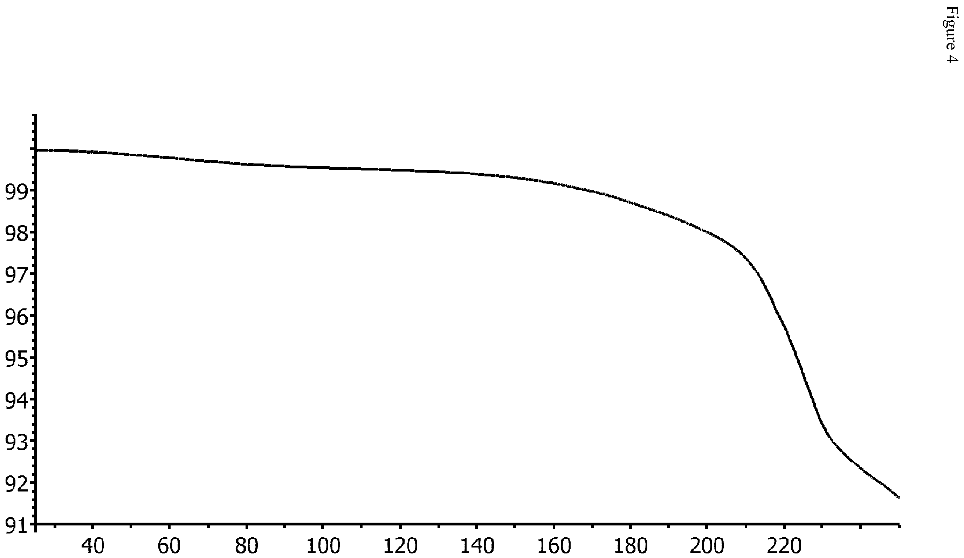

The TGA curve of bictegravir *hemi*-napadisylate shows a mass loss of only about 1.0 w-% in the temperature range of about 25 to 170 °C and a mass loss of only about 2.5 w-% from the start of the measurement at about 25 °C until melting/decomposition starts at about 210 °C. Hence, it can be concluded that neither water nor organic solvents are part of the crystal structure but the mass loss may rather be due to the release of residual solvent/water, which is loosely bound on the surface. Only at temperatures above about 210 °C, where bictegravir *hemi-*napadisylate starts to melt a significant weight loss indicates concomitant decomposition (see also Figure 4 herein).

### Gravimetric moisture sorption

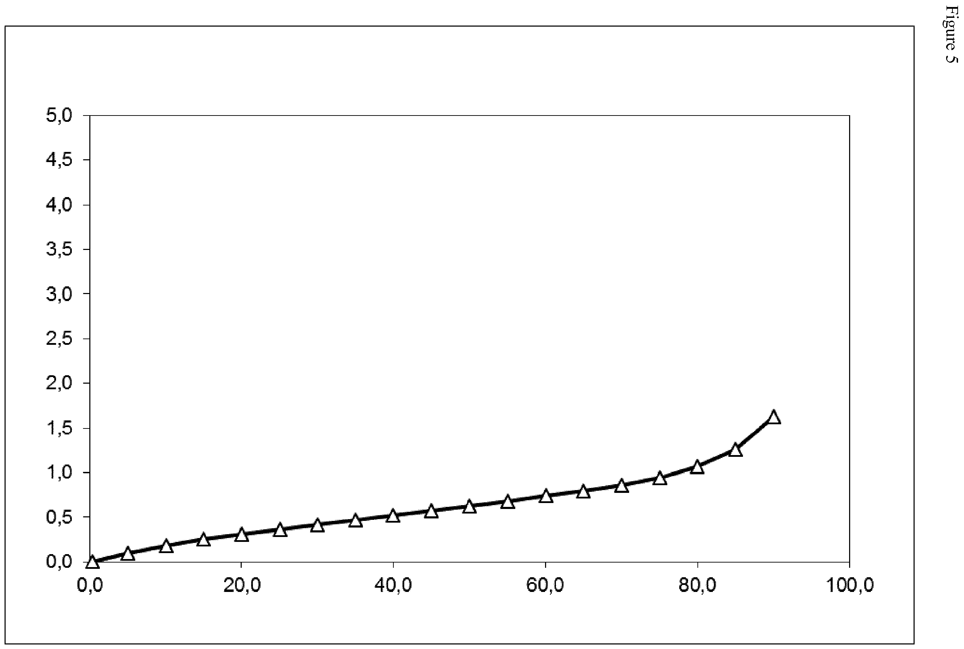

The mass difference in the sorption cycle between 0 and 90% RH was only 1.4 w-% (see also Figure 5 herein). Thereby, a maximum water content of about 1.2 w-% was observed at 90% RH. Hence, bictegravir *hemi*-napadisylate of the present invention can be assigned as being an anhydrous non-hygroscopic salt. After the experiment the sample still showed the same PXRD, indicating that the crystal structure is the same before and after the experiment.

### Electron microscopy

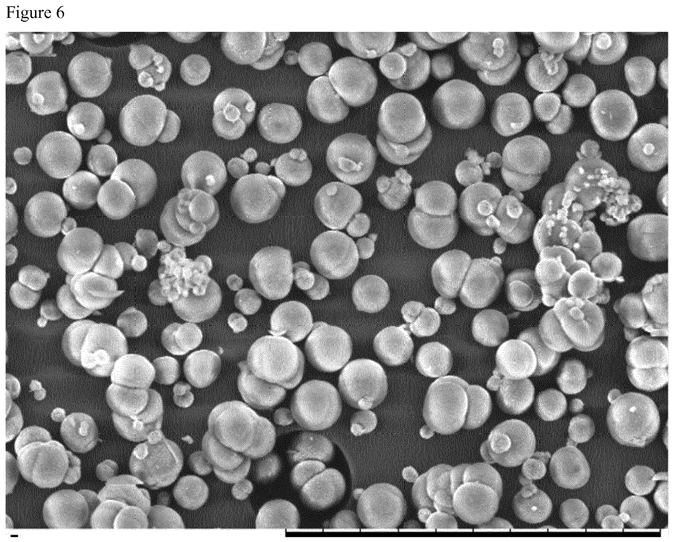

Bictegravir *hemi*-napadisylate of the present invention mainly consists of uniform spherical particles as can also be seen in Figure 6 herein.

### Example 3: Preparation of bictegravir mono-hydrochloride

A reactor was charged with bictegravir (65.0 g, 144.6 mmol, e.g. prepared according to Example 42 of WO 2014/100323 A1) and acetone (750 mL) and the mixture was stirred at 25 °C. Following addition of concentrated hydrochloric acid (assay 37%, 120 mL, 1.5 mol) crystallization occurred and the obtained suspension was further stirred for 1 hour. Then, the temperature with decreased to 0 °C and further stirred at this temperature for 3.5 hours. Finally, the crystals were collected by filtration, washed twice with acetone (each 50 mL) and dried at room temperature under vacuum (30 mbar) for 2 hours to obtain crystalline bictegravir *mono-*hydrochloride (61.4 g). Yield: 87% of theory

### Example 4: Solid-state characterization of bictegravir mono-hydrochloride

### Powder X-ray diffraction

A representative diffractogram of bictegravir mono-hydrochloride is displayed in Figure 7 herein. The corresponding reflection list is provided in Table 3 below.

**Table 3: PXRD reflections of bictegravir mono-hydrochloride in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 6.3 | 16.5 | 21.6 | 26.1 |
| 7.6 | 17.1 | 22.1 | 26.6 |
| 8.9 | 17.5 | 22.6 | 27.0 |
| 13.5 | 18.1 | 22.8 | 27.2 |
| 15.2 | 19.4 | 23.2 | 28.2 |
| 15.3 | 19.9 | 24.1 | 28.9 |
| 15.9 | 21.2 | 24.8 | 29.5 |

### Fourier transform infrared spectroscopy

A representative FTIR spectrum of bictegravir *mono*-hydrochloride according to the present invention is displayed in Figure 8 and the corresponding peak list is provided in Table 4 below.

**Table 2: FTIR peak list of bictegravir mono-hydrochloride according to the present invention; a typical precision of the wavenumbers is in the range of ± 4 cm⁻¹, preferably of ± 2 cm⁻¹.**

| **Wavenumber [cm⁻¹]** | **Wavenumber [cm⁻¹]** | **Wavenumber [cm⁻¹]** |
|---|---|---|
| 2953 | 1391 | 1050 |
| 2863 | 1375 | 1005 |
| 1981 | 1358 | 954 |
| 1911 | 1333 | 893 |
| 1638 | 1300 | 848 |
| 1624 | 1229 | 803 |
| 1603 | 1197 | 789 |
| 1582 | 1167 | 761 |
| 1540 | 1126 | 739 |
| 1459 | 1088 | 696 |
| 1438 | 1067 | 626 |

### Differential scanning calorimetry

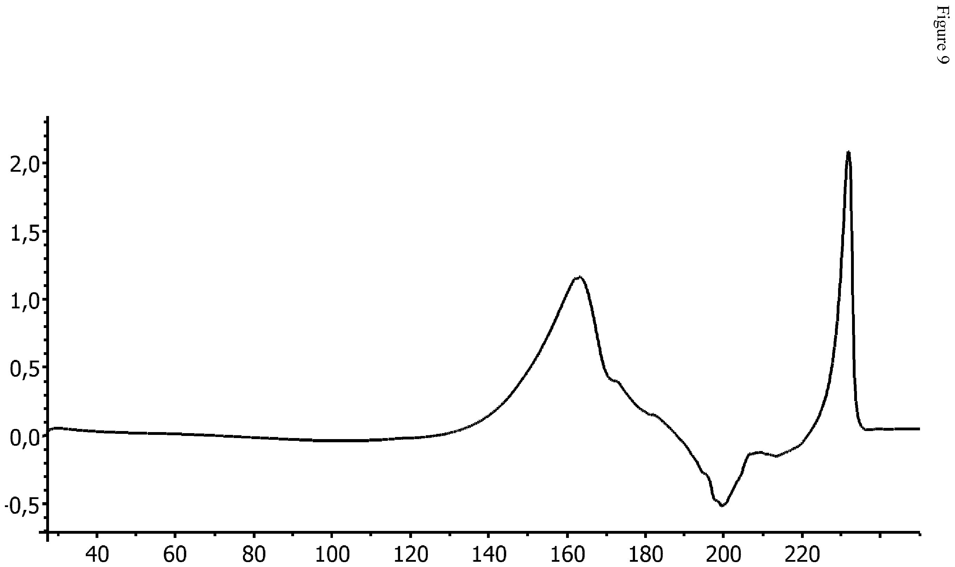

The DSC curve of bictegravir *mono*-hydrochloride shows a first endothermic peak with an onset temperature of about 145 °C, a peak temperature of about 163 °C and an enthalpy of about 115 J/g, which is due to the release of hydrochloric acid from the sample. The exothermic peak, which follows immediately after the first endothermic signal, indicates that some material crystallizes (most probably bictegravir free compound). Finally, a second endothermic signal with an onset temperature of about 228 °C, a peak temperature of about 231 °C and an enthalpy of about 56 J/g occurs, which is most likely due to a melting process. The corresponding DSC curve is displayed in Figure 9 herein.

### Thermo gravimetric analysis

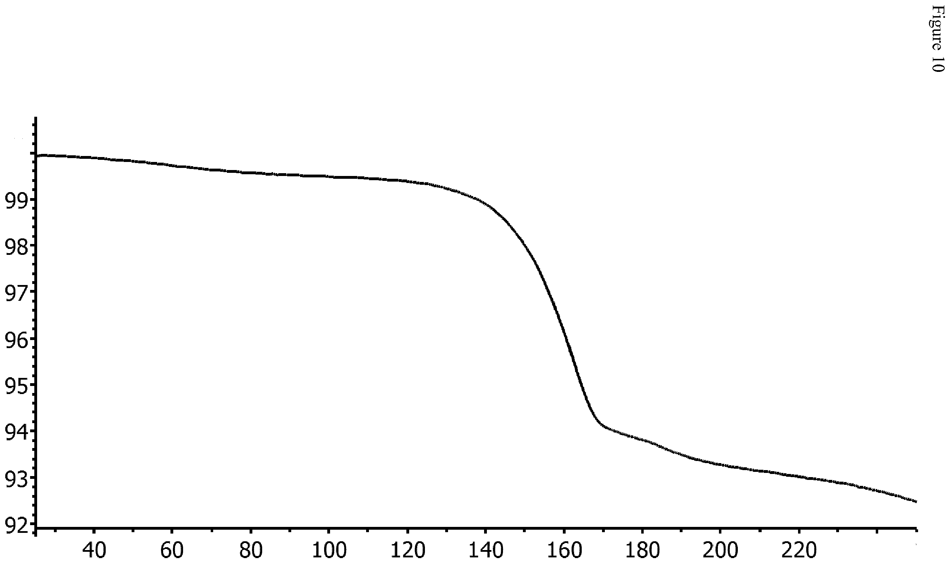

The TGA curve of bictegravir *mono*-hydrochloride shows a first minor mass loss of only about 0.4 w-% in the temperature range of about 25 to 100 °C. Hence, it can be concluded that neither water nor organic solvents are part of the crystal structure but the mass loss may rather be due to the release of residual solvent/water, which is loosely bound on the surface. Thereafter, a second mass loss of about 6.4 w-% in the temperature range of from about 100 to 215 °C occurs, which is due to the loss of hydrochloric acid from the sample. The corresponding TGA curve is displayed in Figure 10 herein.

### Gravimetric moisture sorption

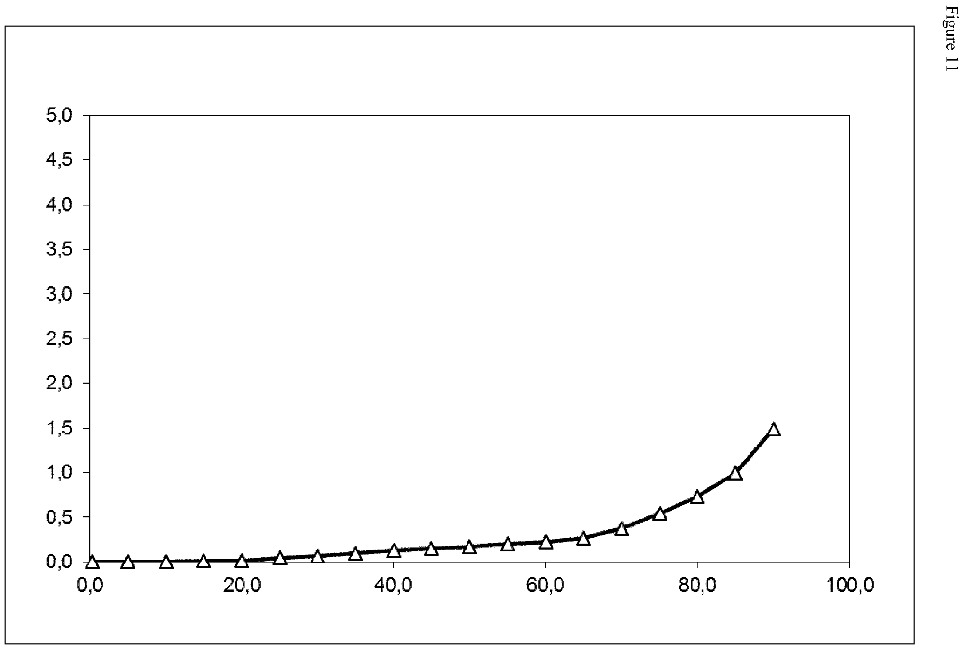

The mass difference in the sorption cycle between 0 and 90% RH was only 0.9 w-% (see also Figure 11 herein). Thereby, a maximum water content of about 0.9 w-% was observed at 90% RH. Hence, bictegravir *mono*-hydrochloride of the present invention can be assigned as being an anhydrous non-hygroscopic salt. After the experiment the sample still showed the same PXRD, indicating that the crystal structure is the same before and after the experiment.

### Electron microscopy

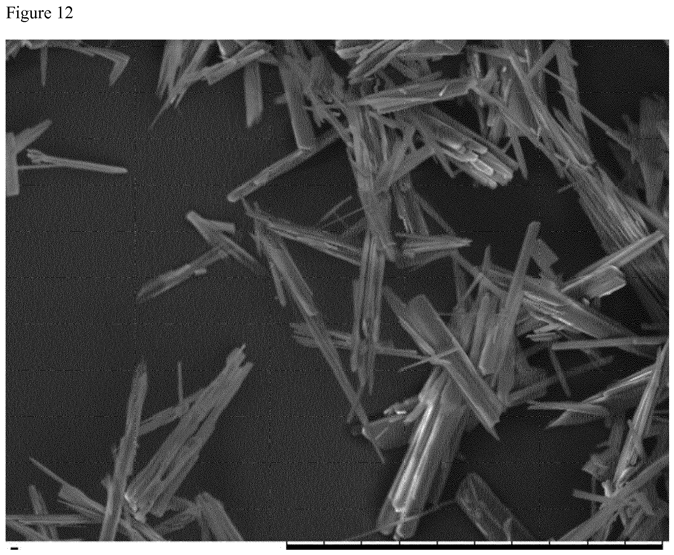

Bictegravir *mono*-hydrochloride of the present invention mainly comprises lath-shaped crystals as can also be seen in Figure 12 herein.

### Example 5: Bilayer tablet comprising bictegravir hemi-napadisylate/emtricitabine/tenofovir alafenamide hemifumarate

| **Ingredient** | **mg/tablet** | |
|---|---|---|
| | first layer | second layer |
| Bictegravir *hemi-*napadisylate | 66.0* | |
| Emtricitabine | | 200 |
| Tenofovir alafenamide hemifumarate | | 28** |
| Microcrystalline cellulose | 246.3 | 113.2 |
| Croscarmellose sodium | 19.4 | 30.2 |
| Magnesium stearate | 4.9 | 5.7 |
| Layer weight | 336.6 | 377.1 |
| **Tablet core weight** | 713.7 | |
| Opadry II | 21 | |
| **Film Coated Tablet** | 734.7 | |

| | | |
|---|---|---|
| *Equivalent to 50 mg bictegravir **Equivalent to 25 mg tenofovir alafenamide | | |

The manufacturing/packaging procedure for the bictegravir *hemi-*napadisylate/emtricitabine/tenofovir alafenamide hemifumarate tablets is divided into five unit processes:
1. mixing bictegravir *hemi*-napadisylate with intergranular excipients, roller compaction or slugging, milling, and blending with extragranular excipients to yield the final powder blend for bictegravir *hemi*-napadisylate
2. mixing emtricitabine and tenofovir alafenamide hemifumarate with intergranular excipients, dry granulation, milling, and blending with extragranular excipients to yield emtricitabine/tenofovir alafenamide hemifumarate powder blend
3. tablet compression to yield bilayer tablet cores
4. tablet film-coating to yield film-coated tablets; and
5. packaging

The manufacturing process steps to produce the final drug product are detailed below.

Bictegravir *hemi*-napadisylate final powder blend:
1. Weigh bictegravir *hemi*-napadisylate and the excipients (microcrystalline cellulose and croscarmellose sodium). Correct the weight of bictegravir *hemi*-napadisylate based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.

Emtricitabine/tenofovir alafenamide hemifumarate final powder blend:
5. Weigh emtricitabine and tenofovir alafenamide hemifumarate and the excipients (microcrystalline cellulose and croscarmellose sodium). Adjust the weight of emtricitabine and tenofovir alafenamide hemifumarate based on their corresponding drug content factors, with a concomitant adjustment to the weight of microcrystalline cellulose.
6. Blend in emtricitabine and tenofovir alafenamide hemifumarate, microcrystalline cellulose and croscarmellose sodium to a tumble blender and blend.
7. Blend in intergranular portion of magnesium stearate to the tumble blender and blend.
8. Dry granulate the resulting blend using roller compactor or slug the resulting blend and mill.
9. Blend in the extragranular portion of magnesium stearate.
Tableting:
10. Compress the final powder blend of bictegravir *hemi*-napadisylate as the first layer and the emtricitabine/tenofovir alafenamide hemifumarate final powder blend as the second layer, with an appropriate main compression force to achieve a target hardness of 17 kP (range: 14 to 20 kP). The final powder blend is compressed to a target layer weight of 323 mg using a target total tablet weight of 700 mg.
Film-coating:
11. Prepare a suspension of Opadry® II. Film-coat the tablet cores to achieve the target tablet weight gain of 3% (range 2-4%). Dry film-coated tablets prior to cooling and discharge.

### Example 6: Film-coated tablet comprising bictegravir hemi-napadisylate

| Ingredient | mg/tablet |
|---|---|
| Bictegravir *hemi-*napadisylate | 66.0* |
| Microcrystalline cellulose | 246.3 |
| Croscarmellose sodium | 19.4 |
| Magnesium stearate | 4.9 |
| **Tablet core weight** | 336.6 |
| Opadry II | 10 |
| **Film Coated Tablet** | 346.6 |

| | |
|---|---|
| *Equivalent to 50 mg bictegravir | |

The manufacturing/packaging procedure for the bictegravir *hemi*-napadisylate tablets is divided into four unit processes:
1. mixing bictegravir *hemi*-napadisylate with intergranular excipients, roller compaction or slugging, milling, and blending with extragranular excipients to yield the final powder blend for bictegravir *hemi*-napadisylate
2. tablet compression to yield tablet cores
3. tablet film-coating to yield film-coated tablets; and
4. packaging

The manufacturing process steps to produce the final drug product are detailed below.

Bictegravir *hemi*-napadisylate final powder blend:
1. Weigh bictegravir *hemi*-napadisylate and the excipients (microcrystalline cellulose and croscarmellose sodium). Correct the weight of bictegravir *hemi*-napadisylate based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.
Tableting:
5. Compress the final powder blend of bictegravir *hemi*-napadisylate with an appropriate main compression force to achieve a target hardness of 17 kP (range: 14 to 20 kP). The final powder blend is compressed to a target total tablet weight of 323 mg.
Film-coating:
6. Prepare a suspension of Opadry® II. Film-coat the tablet cores to achieve the target tablet weight gain of 3% (range 2-4%). Dry film-coated tablets prior to cooling and discharge.

### Example 7: Bilayer tablet comprising bictegravir mono-hydrochloride/emtricitabine/tenofovir alafenamide hemifumarate

| **Ingredient** | **mg/tablet** | |
|---|---|---|
| | first layer | second layer |
| Bictegravir *mono-*hydrochloride | 54.1* | |
| Emtricitabine | | 200 |
| Tenofovir alafenamide hemifumarate | | 28** |
| Microcrystalline cellulose | 246.3 | 113.2 |
| Croscarmellose sodium | 19.4 | 30.2 |
| Magnesium stearate | 4.9 | 5.7 |
| Layer weight | 324.7 | 377.1 |
| **Tablet core weight** | 701.8 | |
| Opadry II | 21 | |
| **Film Coated Tablet** | 722.8 | |

| | | |
|---|---|---|
| *Equivalent to 50 mg bictegravir **Equivalent to 25 mg tenofovir alafenamide | | |

The manufacturing/packaging procedure for the bictegravir *mono-*hydrochloride/emtricitabine/tenofovir alafenamide hemifumarate tablets is divided into five unit processes:
1. mixing bictegravir *mono*-hydrochloride with intergranular excipients, roller compaction or slugging, milling, and blending with extragranular excipients to yield the final powder blend for bictegravir *mono*-hydrochloride
2. mixing emtricitabine and tenofovir alafenamide hemifumarate with intergranular excipients, dry granulation, milling, and blending with extragranular excipients to yield emtricitabine/tenofovir alafenamide hemifumarate powder blend
3. tablet compression to yield bilayer tablet cores
4. tablet film-coating to yield film-coated tablets; and
5. packaging

The manufacturing process steps to produce the final drug product are detailed below.

Bictegravir *mono*-hydrochloride final powder blend:
1. Weigh bictegravir *mono*-hydrochloride and the excipients (microcrystalline cellulose and croscarmellose sodium). Correct the weight of bictegravir *mono*-hydrochloride based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.

Emtricitabine/tenofovir alafenamide hemifumarate final powder blend:
5. Weigh emtricitabine and tenofovir alafenamide hemifumarate and the excipients (microcrystalline cellulose and croscarmellose sodium). Adjust the weight of emtricitabine and tenofovir alafenamide hemifumarate based on their corresponding drug content factors, with a concomitant adjustment to the weight of microcrystalline cellulose.
6. Blend in emtricitabine and tenofovir alafenamide hemifumarate, microcrystalline cellulose and croscarmellose sodium to a tumble blender and blend.
7. Blend in intergranular portion of magnesium stearate to the tumble blender and blend.
8. Dry granulate the resulting blend using roller compactor or slug the resulting blend and mill.
9. Blend in the extragranular portion of magnesium stearate.
Tableting:
10. Compress the final powder blend of bictegravir *mono*-hydrochloride as the first layer and the emtricitabine/tenofovir alafenamide hemifumarate final powder blend as the second layer, with an appropriate main compression force to achieve a target hardness of 17 kP (range: 14 to 20 kP). The final powder blend is compressed to a target layer weight of 323 mg using a target total tablet weight of 700 mg.

Film-coating:
11. Prepare a suspension of Opadry® II. Film-coat the tablet cores to achieve the target tablet weight gain of 3% (range 2-4%). Dry film-coated tablets prior to cooling and discharge.

### Example 8: Film-coated tablet comprising bictegravir mono-hydrochloride

| Ingredient | mg/tablet |
|---|---|
| Bictegravir *mono-*hydrochloride | 54.1* |
| Microcrystalline cellulose | 246.3 |
| Croscarmellose sodium | 19.4 |
| Magnesium stearate | 4.9 |
| **Tablet core weight** | 324.7 |
| Opadry II | 10 |
| **Film Coated Tablet** | 334.7 |

| | |
|---|---|
| *Equivalent to 50 mg bictegravir | |

The manufacturing/packaging procedure for the bictegravir *mono*-hydrochloride tablets is divided into four unit processes:
1. mixing bictegravir *mono*-hydrochloride with intergranular excipients, roller compaction or slugging, milling, and blending with extragranular excipients to yield the final powder blend for bictegravir *mono*-hydrochloride
2. tablet compression to yield tablet cores
3. tablet film-coating to yield film-coated tablets; and
4. packaging

The manufacturing process steps to produce the final drug product are detailed below.

Bictegravir *mono*-hydrochloride final powder blend:
1. Weigh bictegravir *mono*-hydrochloride and the excipients (microcrystalline cellulose and croscarmellose sodium). Correct the weight of bictegravir *mono*-hydrochloride based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.
Tableting:
5. Compress the final powder blend of bictegravir *mono*-hydrochloride with an appropriate main compression force to achieve a target hardness of 17 kP (range: 14 to 20 kP). The final powder blend is compressed to a target total tablet weight of 323 mg.
Film-coating:
6. Prepare a suspension of Opadry® II. Film-coat the tablet cores to achieve the target tablet weight gain of 3% (range 2-4%). Dry film-coated tablets prior to cooling and discharge.

## Claims

1. A salt comprising bictegravir and 1,5-naphthalenedisulfonic acid.

2. The salt of claim 1 **characterized by** the chemical structure according to Formula (II) 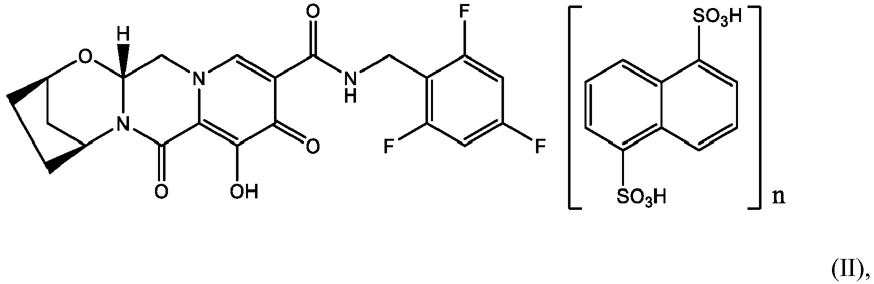 wherein n is in the range of from 0.4 to 0.6.

3. The salt as defined in claim 1 or 2 **characterized in that** the salt is crystalline.

4. The salt as defined in any one of the preceding claims **characterized by** having a powder X-ray diffractogram comprising reflections at 2-Theta angles of (7.2 ± 0.2)°, (7.8 ± 0.2)° and (23.5 ± 0.2)°, when measured at a temperature in the range of from 20 to 30 °C with Cu-Kalpha_{1,2} radiation having a wavelength of 0.15419 nm.

5. The salt of claim 4 **characterized by** having a powder X-ray diffractogram comprising additional reflections at 2-Theta angles of (20.1 ± 0.2)° and (21.0 ± 0.2)°, when measured at a temperature in the range of from 20 to 30 °C with Cu-Kalpha_{1,2} radiation having a wavelength of 0.15419 nm.

6. A process for preparing the salt comprising bictegravir and 1,5-naphtalenedisulfonic acid as defined in any one of claims 1 to 5 comprising:
(e) reacting bictegravir and 1,5-naphtalenedisulfonic acid in the presence of a suitable solvent;
(f) allowing for the crystallization of the salt;
(g) separating at least a part of the crystals obtained in step (b);and
(h) drying the crystals obtained in step (c).

7. A salt comprising bictegravir and hydrochloric acid.

8. The salt of claim 7 **characterized by** the chemical structure according to Formula (III) 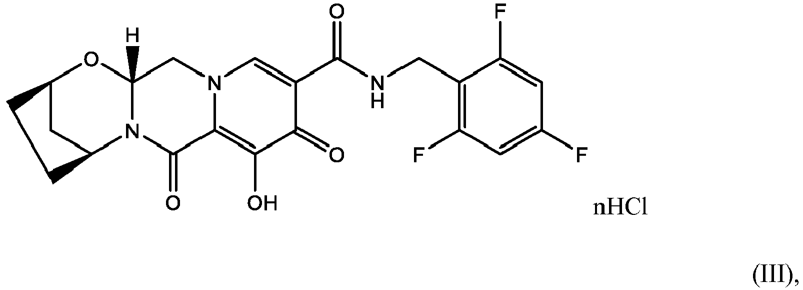 wherein n is in the range of from 0.8 to 1.2.

9. The salt as defined in claim 7 or 8 **characterized in that** the salt is crystalline.

10. The salt as defined in any one of claims 7 to 9 **characterized by** having a powder X-ray diffractogram comprising reflections at 2-Theta angles of (8.9 ± 0.2)°, (16.5 ± 0.2)° and (18.1 ± 0.2), when measured at a temperature in the range of from 20 to 30 °C with Cu-Kalpha_{1,2} radiation having a wavelength of 0.15419 nm.

11. A process for preparing the salt comprising bictegravir and hydrochloric acid as defined in any one of claims 7 to 10 comprising:
(e) reacting bictegravir and hydrochloric acid in the presence of a suitable solvent;
(f) allowing for the crystallization of the salt;
(g) separating at least a part of the crystals obtained in step (b);and
(h) drying the crystals obtained in step (c).

12. Use of a salt as defined in any one of claims 1 to 5 or claims 7 to 10 for the preparation of a pharmaceutical composition.

13. A pharmaceutical composition comprising the salt as defined in any one of claims 1 to 5 or the salt as defined in any one of claims 7 to 10, and at least one pharmaceutically acceptable excipient.

14. The pharmaceutical composition of claim 13, wherein the one or more additional active pharmaceutical ingredients are selected from emtricitabine and tenofovir.

15. The pharmaceutical composition as defined in any one of claims 13 to 14, wherein the pharmaceutical composition is an oral solid dosage form, in particular for use in the treatment and/or prophylaxis of HIV-1 infections.
